# EUROPEAN PATENT APPLICATION

(11) **EP 2 361 546 A2**
(43) Date of publication of application: **31.08.2011**
(21) Application number: 11153493.9
(22) Date of filing: 14.04.2005
(51) Int. Cl.: A61B 1/06, A61B 1/24

(54) **Systems and methods for detection of disease including oral scopes and ambient light management systems (ALMS)**

(30) Priority: 14.04.2004 US 562469 P; 12.10.2004 US 618287 P; 16.12.2004 US 16567
(62) Divisional of application: 05735631.3
(71) Applicant: LED MEDICAL DIAGNOSTICS, INC., White Rock BC V4B 1C5 (CA)
(72) Inventor: Gilhuly, Terence J., Vancouver British Columbia V6K 3J2 (CA); Whitehead, Peter, West Vancouver British Columbia V7V 3X5 (CA)
(74) Representative: Ferreccio, Rinaldo

(57) **Abstract**

Methods and systems related to detecting disease, such as oral cancer, in a patient, using a viewing scope to investigate a patient's tissues. The systems and methods excite and detect fluorescence from the tissue. The fluorescence can then be evaluated, and the possibility of certain diseases such as cancer can be determined. The devices include an ambient light management system (ALMS, often referred to as a "vestibular device") that manages background light in the health practitioner's office. This device can be used with scope systems for fluorescence based detection of abnormal tissue.

## Description

### CROSS-REFERENCE TO RELATED APPLICATIONS

The present application claims priority from United States provisional patent application No. 60/562,469 filed April 14, 2004; from United States provisional patent application No. 60/618,287 filed October 12, 2004; and, from United States patent application No. 11/016,567 filed December 16, 2004, all of which are incorporated herein by reference in their entirety and for all teachings, disclosures and purposes.

### BACKGROUND

Detection of cancer, including precancerous and early cancerous cells, has always been a difficult and uncertain process. One of the approaches to identifying cancerous cells has been to measure the cells' autofluorescence signature, because cancerous cells have a distinct autofluorescence signature relative to healthy cells. Other diseases also have changes in their autofluorescent signature. However, the detection of autofluorescence in traditional dental and medical environments has been problematic because the autofluorescence signature itself is very very small compared to the ambient light typically present in an examination room, operating room, etc.

The following are some references relating to the detection of cancer in the oral cavity. As with all other references cited herein, including in the Cross Reference to Related Applications, these references are incorporated herein in their entirety for all their teachings and for all purposes. The inclusion of such references herein does not indicate that any of the references either are, or aren't, prior art to the instant application. Zheng, W., et al., Detection of squamous cell carcinomas and pre-cancerous lesions in the oral cavity by quantification of 5-aminolevulinic acid induced fluorescence endoscopic images, Lasers Surg. Med. 31:151-157, 2002; Utzinger U, et al., Optimal visual perception and detection of oral cavity neoplasia, Cancer 2003 Apr 1;97(7):1681-92; Muller, MG, et al., Spectroscopic detection and evaluation of morphologic and biochemical changes in early human oral carcinoma, Cancer 2003;97:1681-92; Majumder, S., Nonlinear pattern recognition for laser-induced fluorescence diagnosis of cancer, Lasers Surg. Med. 33:48-56, 2003; Tsai, T., et al., In vivo autofluorescence spectroscopy of oral premalignant and malignant lesions: Distortion of fluorescence intensity by submucous fibrosis, Lasers Surg Med 2003;32(1):17-24; Ebihara, A, Detection and diagnosis of oral cancer by light-induced fluorescence, Lasers Surg. Med. 32:17-24, 2003, PMID: 12516066; Zheng W, Detection of neoplasms in the oral cavity by digitized endoscopic imaging of 5-aminolevulinic acid-induced protoporphyrin IX fluorescence, Int J Oncol 2002 Oct, 21(4):763-8; PMID: 12239614; Wang CY, Autofluorescence spectroscopy for in vivo diagnosis of DMBA-induced hamster buccal pouch pre-cancers and cancers, J Oral Pathol Med 2003 Jan;32(1):1.8-24, PMID: 12558954; Onizawa K, Characterization of autofluorescence in oral squamous cell carcinoma, Oral Oncol 2003 Feb;39(2):150-6, PMID: 12509968;

Accordingly, there has gone unmet a need to improve the ability of a doctor, dentist or other person to diagnose cancerous or precancerous cells in a target tissue in a typical medical or dental setting. The present invention provides these and other advantages.

### SUMMARY

The present innovation relates to detecting disease, such as oral cancer, in a patient, using a viewing scope to investigate a patient's tissues. In some embodiments, the systems and methods excite and detect fluorescence from the tissue. The fluorescence can then be evaluated, and the possibility of certain diseases such as cancer can be determined. The devices include an ambient light management system (ALMS, often referred to as a "vestibular device"): a system for the management of background light in the health practitioner's office. This device can be used with such scope systems for fluorescence based detection of abnormal tissue. The current discussion also pertains to the detection of abnormal oral tissues, and to various elements of the systems and methods. The discussion includes the patient being diagnosed and the health practitioner, be it dentist, general practitioner or hygienist, administering the test.

Accordingly, the present innovations relate to methods and systems, etc., of detecting abnormal tissues in the mouth and other body orifices and other locations through viewing and measurement of the tissue's fluorescent or other light-based properties. This can be done in a dentist or doctor's office under normal lighting conditions. Since fluorescence produced by tissue (autofluorescence) is of very low light power, fluorescence based abnormal tissue detection is preferably performed in very low levels of background or ambient light so as to not interfere with the measurement. Therefore, methods and systems for preventing ambient light from entering and absorbing stray light that does enter the viewing field is provided for proper operation of the system.

Solutions include vestibular devices such as veils, bibs and masks. The vestibular devices sit on or are otherwise attached to the patient's face or head area, for example below the eyes so that the examination illumination is not directed into the patient's eyes, and covering the mouth. If desired, the vestibular device can be attached to the chair or other available structures with or without attachment to the patient him or her self, and then the patient can, if desired, be provided with protective eyewear.

In appropriately sized embodiments, a porthole or slit is positioned at the mouth through which the practitioner can pass the scope. The veil can be held to the patient's head or other body part through elastic straps or strings at the rear of their head or behind their ears, ear hooks or some other comparable method or system. There can also be a piece of aluminum or other malleable material at the nose for conformity with the patient's face. In other embodiments, the practitioner, and/or the patient, are included inside the veil or other vestibular element.

Exemplary procedures include the following:
1. The patient sits upright in a chair during the procedure. He/she will typically not be in a supine position, although such can be used if desired. The supine position causes the patient's tongue to fall back into the throat restricting tissue exposure.
2. The light source, scope, etc., preferably moves freely without the vestibular device either substantially constraining the movement or occluding the view of the dentist or other practitioner.
3. The practitioner may, if desired, be able to grab and move the tongue without introducing stray light and without disrupting the procedure.

Exemplary device constructions include the following:
4. The vestibule device can be composed of a draping material or other suitable shroud material, which can rigid or floppy or in-between as desired and depending on the given embodiment, non-rigid devices such as internal stiffeners can be included for shape. Systems and methods of attachment to the viewing scope, to a separate light source (if any), and to a tool (if any). Systems and methods of attachment to the patient can also be included.
   a. Attachment of the vestibule device to the scope: The scope or other investigative device enters the vestibular device to become, in some embodiments, a part of the ALMS, through a port or hole in the drape, typically in-line with the mouth or other target tissue. An attachment and/or seal enhances darkness and confident manipulation of the vestibular device and other elements of the systems without introduction of unwanted light.
   b. Attachment of the vestibule device to the patient. A malleable/shapeable piece of material that conforms to the curves of the nose and cheeks can be provided, such as an elastic that goes around the patients head or ear clips similar to frames of glasses. Attachment to the patient's head can allow the dentist to have more freedom to move the device to different angles. Molding around the nose and cheeks acts as a seal that improves the reduction of ambient light.
5. In some embodiments the vestibular device is disposable to inhibit the spread of bacteria, viruses or material between patients.
6. Particularly in embodiments where the vestibular device may be disposable, it should be inexpensive.

Exemplary materials for the vestibular device include the following:
7. The draping material can be any desired, generally opaque material, such as a nonwoven synthetic. The material can also be more than one sheet of a given material, or other combinations of materials.
8. The particular composition of the draping material may be determined by the draping properties necessary to cover the patient comfortably without impeding workflow of the physician.
9. The material from which the ALMS is made preferably meets guidelines for flammability safety.
10. For patient health concerns, the materials preferably pass cytotoxicity and skin irritation tests for short term exposures.
11. Materials are preferably not pyrogenic/allergenic.
12. The materials are preferably non-PVC to permit use in Europe.
13. So as to not interfere or modify the measurement made in some embodiments of investigations, materials preferably do not fluoresce or at least produce substantially less fluorescence than a typical abnormal tissue measurement within the visible light spectrum.
14. Materials are preferably sufficiently opaque such that external light in a normally lit (e.g., normal reading and viewing conditions) dentist/doctor's office will not substantially influence viewing of tissue fluorescence or other desired examination/response light.
15. Surface materials can be dark in color (e.g., black, forest green or navy blue) and non-reflective to better the measurements by absorbing stray light that does get through and/or under the ALMS.

Exemplary dimensions for the vestibular device include the following:
16. The vestibular devices are preferably not an encumbrance to the patient and the practitioner; e.g., does not interfere with movement during the procedure.
17. The vestibular device is typically large enough that it will drape over the patient's mouth, and possibly body, and block out light that could enter at various points such as at the shoulders (in embodiments where the shoulders are of concern). The vestibular device, which can be disposable in whole or in part, is large enough to prevent light from entering when the device is moved during the procedure.
18. The vestibular device will, in some embodiments, be attached to the patient and thereby in certain embodiments should not have significant perceptible or uncomfortable weight, for example less than 100g.

Exemplary interfacing for the vestibular device include the following:
19. The vestibular device can couple/integrate with the investigative device such that it does not fall away from the investigative device unintentionally.
20. The vestibular device can comprise a port or other access point of allowing the investigating device to access the patient's mouth or other body part under investigation.
21. The vestibular device can interface to the patient by being formed to make a seal with his/her face or other body part. In one embodiment this is accomplished via a malleable/shapeable piece of material in the mask.
22. The draping material may be large enough and flexible enough that the health practitioner can grab and manipulate the tongue or other body from beneath the draping material without introducing ambient light.
23. Space is typically created between the patient and the light source. To do this, for example, the draping material can be relatively stiff, reinforced with another material, more of the same material and/or stitching or will have a brim or other space-making structure.

In these and other embodiments (unless expressly stated otherwise or clear from the context), all embodiments, aspects, features, etc., of the innovations herein can be mixed and matched, combined and permuted in any desired manner.

These and other aspects, features and embodiments are set forth within this application, including the following Detailed Description and attached drawings. In addition, various references are set forth herein, including in the Cross-Reference To Related Applications, that discuss certain systems, apparatus, methods and other information; all such references are incorporated herein by reference in their entirety and for all their teachings and disclosures, regardless of where the references may appear in this application.

### BRIEF DESCRIPTION OF THE DRAWINGS

FIG. 1 depicts a schematic view of an ALMS (ambient light management system) configured for medical use.

FIG. 2 depicts a side schematic view of an ALMS according to the present invention.

FIGS. 3 and 4 depict an embodiment substantially similar to the embodiment in FIG. 2 accept that the shroud is substantially black and the device is configured to rest below the eyes of the patient.

FIGS. 5 and 6 depict a schematic front and side view of an ALMS comprising a brim.

FIG. 7 is a schematic elevated side view depicting an embodiment of the ALMS suitable for use with a supine patient.

FIG. 8 depicts a schematic view of a vestibular device in the form of a bib.

FIG. 9 depicts a schematic representation of a device comprising a brow and ear pieces.

FIGS. 10-12 schematically depict various embodiments of seals suitable for use to retain the ALMS to a patient.

FIGS. 13-13A depict a variety of different shapes that can be used for the port for the viewing scope, the illumination light, the tool or the otherwise as desired.

FIG. 14 schematically depicts a port key that removable attaches to the viewing scope.

FIGS. 15A-C depicts schematic views of a port key that can be used to connect a viewing scope to the substantially opaque vestibular device of the ALMS.

Figure 16 depicts schematically another embodiment of a vestibular device.

Figure 17 depicts a top schematic view of various shapes that can be used with the vestibular device to create the vestibular space.

Figure 18 depicts a plurality of different shapes that can be used with brow pieces or other space-making vestibular devices.

Figure 19 depicts battens suitable for use in a brow piece of a vestibular device.

Figure 20 depicts further embodiment for attaching the viewing scope and the vestibular device.

Figure 21 is a side view of a viewing scope attached to the multi-layer shroud and port depicted in Figure 20.

Figures 22 A-C depict a key way and tube port 151 in a shroud along with a corresponding distal end of a viewing scope.

Figure 23 depicts one embodiment before the manufacturer of a shroud 68 such as depicted in Figure 22A.

Figure 24 depicts an elevated front plan view of a surgical mask-type embodiment of a shroud comprising the port.

Figure 25 depicts a close up front plan view of the shroud and port of Figure 24.

Figure 26 depicts a cutaway side view of a docking mechanism of a vestibular device such as depicted in Figures 24 and 25.

Figures 27-28 depict a front plan view of a twist-and-lock configuration of a slide-in port.

Figure 29 depicts close up side view port and a vestibular device comprising multiple layers of fabric and having differently shaped passages to form an interference-fitting port for a viewing scope.

FIG. 30 is an elevated perspective view of a viewing scope according to one embodiment of the innovations herein.

FIG. 31 depicts a partial element side view of a viewing scope.

FIG. 32 depicts a cut-away side view of a viewing scope.

FIG. 33 depicts a cut-away side view of the head of the viewing scope depicted in FIGS. 34-43 depict schematic side, cut-away views of a variety of connection mechanisms to connect the various devices in the system.

FIG. 44 provides a further embodiment of an adapter wherein both a power source and a light source are maintained within the adapter itself.

FIG. 45 depicts a cut-away view of a hand-held embodiment of a viewing scope wherein the viewing scope comprises a light source and power source in the handle.

FIG. 46 depicts a perspective view of a schematic figure of an ALMS.

FIG. 47 depicts a top perspective view of a viewing scope with a conduit detachable from an external power and light source.

FIG. 48 depicts a viewing scope connected via a conduit and plug-in adapter to an external power and light source.

FIG. 49 depicts a tool 288 comprising an extension 290 having a ridge 300 configured to attach to the distal end of a viewing scope.

FIG. 50 is an elevated side view of the device of FIG. 49.

FIG. 51 depicts an exploded perspective view of a viewing scope.

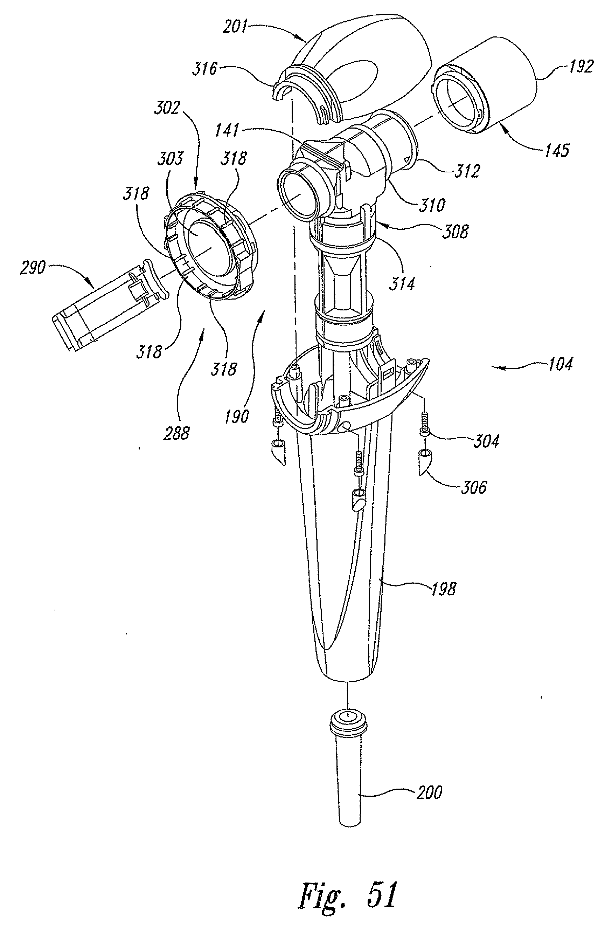

FIG. 51 depicts an exploded view of a viewing scope.

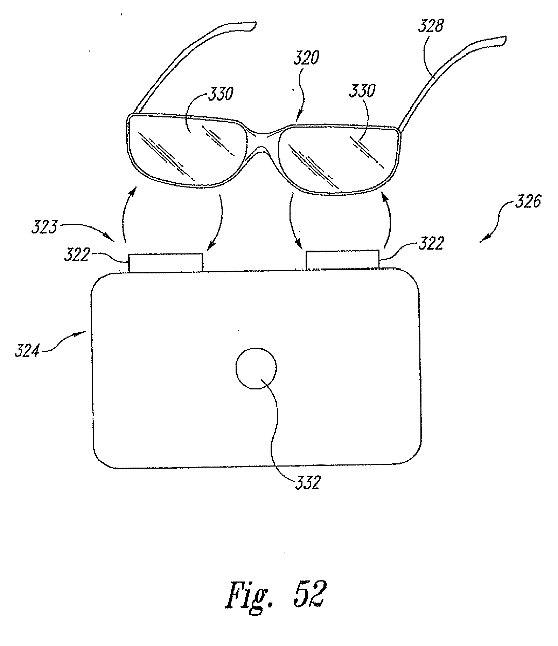

FIG. 52 depicts a binocular embodiment of an ALMS according to the innovations herein.

### DETAILED DESCRIPTION

The present innovation is directed to viewing scopes, in some embodiments referred to herein as "Velscopes," suitable for examination of light such as fluorescent light emanating from a patient. In some embodiments the innovations are directed to viewing scopes for the detection of lesions such as cancer in an oral cavity or vaginal area. Typically, the viewing scopes are configured for the detection of fluorescence or other very faint light emanations from a target tissue and are used in combination with an ALMS such as a shroud or vestibule that substantially covers the target area and substantially prevents any and all undesired light, such as ambient room light, from entering the viewing area.

Also typically, the vestibule comprises at least one port sized and configured to operably attach to the scope, usually at the distal end (or to one or more devices attached to the scope and extending between the scope and the shroud). Various embodiments of the scope and shroud are depicted and discussed herein.

### DISCUSSION OF THE FIGURES

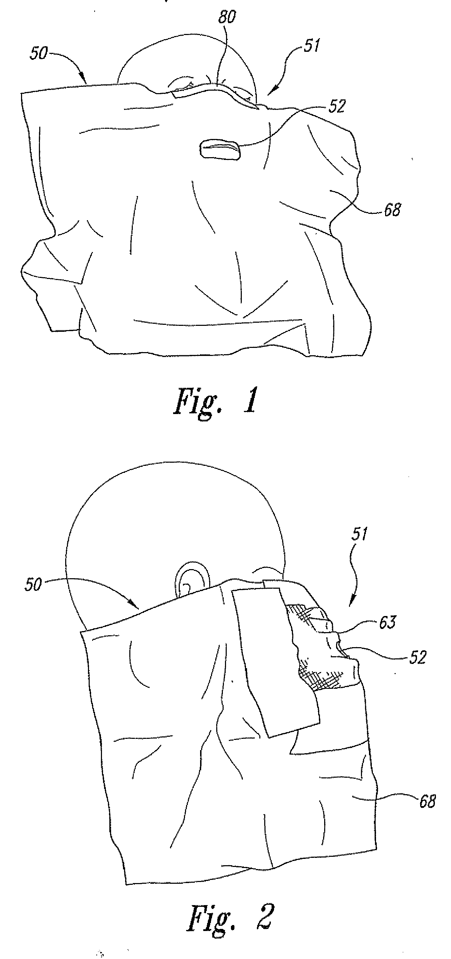

FIG. 1 depicts a schematic view of an ALMS (ambient light management system) configured for health practitioner use. Briefly, the ALMS is configured to fit about a portion of a body of a patient (the oral cavity in FIG. 1) comprising a target tissue such as the tongue, cheeks or gums in the embodiment shown, or such as cervical, vaginal, auditory or epidermal tissue in other embodiments, and even tissue laid open via inspection during surgery, particularly if such tissues are suspected of containing cancer-related cells or tissues, such as neoplasias, malignancies, benign tumors, dysplasias, etc.

In FIG.1, the ALMS 50 comprises a port 52 and a shroud or drape 68. The shroud or drape is fixed to block substantially all ambient light from reaching the target tissue and is configured to form a vestibule that covers at least the portion of the body that is under inspection. Further, the shroud 68 is sized and configured to allow at least one hand of a user and/or a tool manipulated by the user to touch the portion of the body within the vestibule under inspection. Such a tool indicates an actual physical device, such as a mechanical projection that extends from the distal portion, or even the distal tip of the viewing scope, but could also be a mechanical device that extends into the interrogation area via a separate port and/or via the viewing port even though not attached to the viewing scope. Such a tool does not include things that emanate from the viewing scope such as light or electricity. As noted, the vestibular device 50 of the ambient light management system (ALMS) 51 further comprises a port 52 located to provide visual access to the target and configured to substantially opaquely join a viewing scope configured to operably connect a viewer to the target under inspection. As depicted in other figures discussed elsewhere herein, the ALMS in certain embodiments further comprises a light source configured to provide light, such as excitation light for fluorescence, to the target. In certain desired embodiments, the light source can provide a plurality of different forms of light, such as excitation light, regular white light, IR, light, photodynamic treatment (PDT) light, etc.

ALMS 51 also comprises a seal 80 which in the embodiment shown is a thin, lightweight metal strip that can be formed about the bridge of the nose (either above or below the eyes) of the patient to substantially inhibit light from entering the target area from the eye region of the patient.

FIG. 2 depicts an ALMS 51 comprising a vestibular device 50 having a port 52. In the embodiment in FIG. 2, the drape 68 has been combined with an operating room (OR) mask 63. The OR mask also provides fixation mechanisms (not shown) that simplify the attaching the ALMS 51 to a patient.

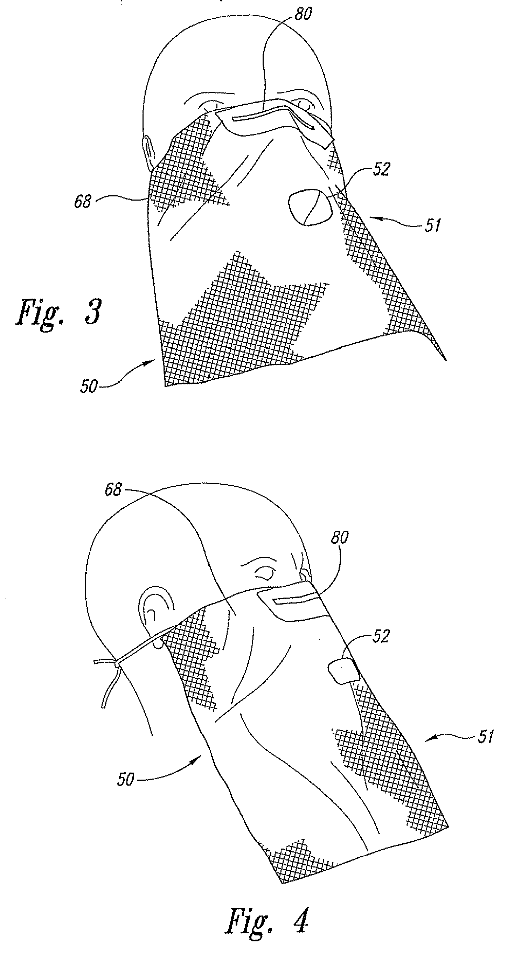

FIGS. 3 and 4 depict an embodiment substantially similar to the embodiment in FIG. 2 except that the shroud 68 is substantially black and the device is configured to rest below the eyes of the patient. In this embodiment, the drape or shroud 68 comprises two squares of medical grade polypropylene (in one embodiment, the weight can be 1.91oz/yard²) joined together. Also attached are an elastic band and a formable metal strip 80 that assist in keeping the mask in place about the face of the patient and in preventing undesired light from reaching the target area. As noted elsewhere herein, in this and most other embodiments, the material is dark and further is non-fluorescing nor reflective such that substantially all light within the interrogation are, in certain embodiments consists substantially only of whatever illumination/excitation light is provided by the user and any auto fluorescence, Raman or other light that is emanated from the patient.

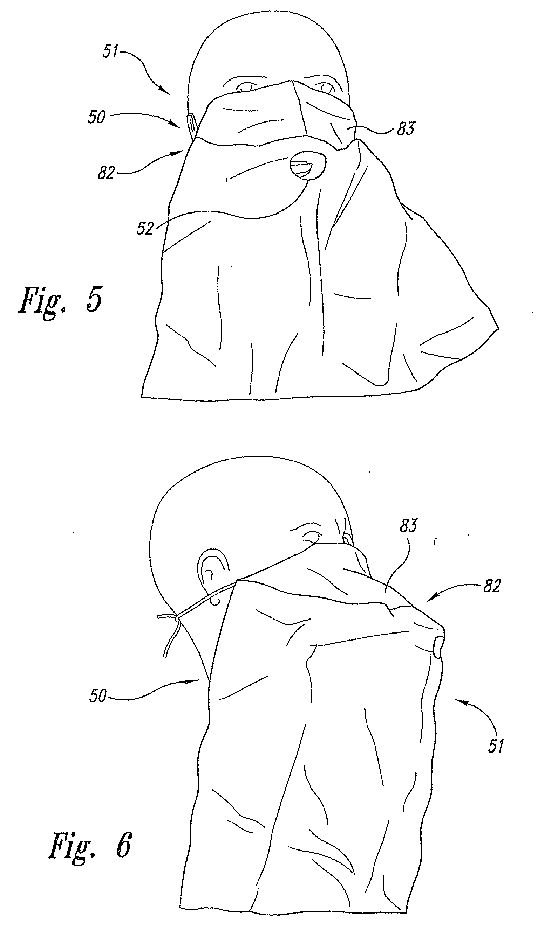

FIGS. 5 and 6 depict schematic views of an ALMS as discussed herein, wherein the ALMS further comprises a brow piece 82 that forms a brim 83. As depicted, the brow piece 82 and brim 83 is substantially stiff (in one embodiment the brim can be shaped substantially similarly to an OR duck bill-type, N95 mask), cut to any desired shape. The brow piece 82 enhances the size and rigidity of the vestibule area within the vestibular device 50 of ALMS 51.

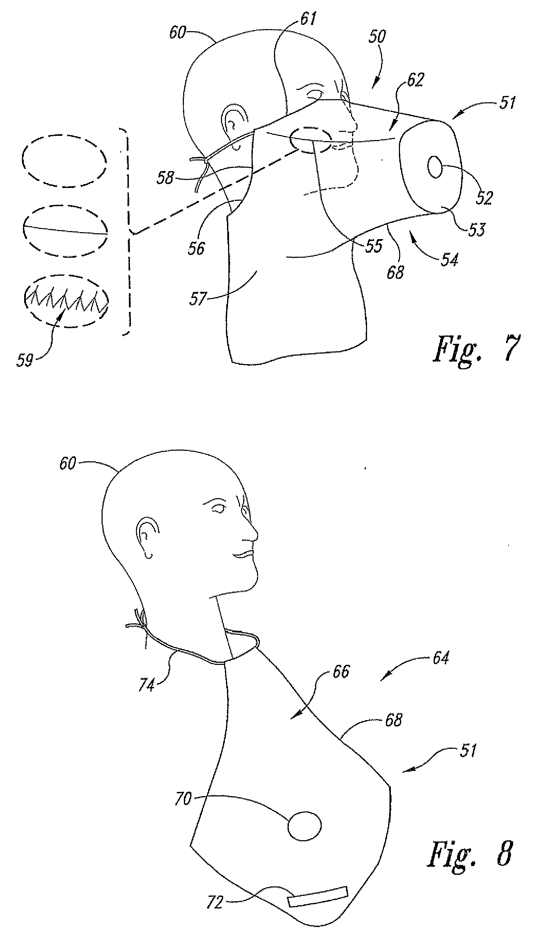

FIG. 7 depicts a vestibular device 50 for an ALMS 51. As depicted, the vestibular device 50 comprises a port 52 sized and configured for the light, scope, tool, etc., and comprises a space-making top shape 53. In the embodiments shown, the space-making top sheet 53 is substantially circular but any desired configuration such as oval, square, hexagonal, octagonal, etc., can be used. Due to the top shape 53, stiffeners (e.g., rod-like, substantially stiff members which can, if desired, join together to forum a cage) form a tube 54 through which the user can see using the scope (not shown) and within which the user can manipulate the target under inspection if desired. Stiffeners 55 can be seen within cutaway and can be formed in any desired shape or configuration. Moreover, the material can be configured to have no seams, or accordion stitching to assist in maintaining the shape of the ALMS 51 if desired. The vestibular device 50 further comprises an open end 56 that is placed around the target in question, which, as noted elsewhere herein, in the instant embodiment is an oral cavity of a patient, but could be any other desired cavity such as an area suitable for colposcopic examination, dermal site, or even the interior or exterior of a surgical site.

The length 57 of the vestibular device 50 can be any desired length provided it is adequate to provide the desired blocking of undesired light such as ambient light. If desired, the vestibular device 50 can further comprise an adhesive or other sticky or adhering substance to aid the vestibular device and adhering to the patient 60. Moreover, the edges of the vestibular device (and other portions if desired) can be formed of a shapable material 61 and of course the material 62 of the drape 68 reduces light transmission and creation within the vestibular space. The seams and rod structures can, if desired be accordion shaped 59.

FIG. 8 depicts a schematic view of a vestibular device 64 comprising a substantially opaque material 66 to form a drape 68 comprising a port 70 wherein the vestibular device 50 is shaped in the form of a bib. The bib comprises an attachment element 72, for example an adhesive or other adhering composition that is on the external side the bib when the bib is hanging as depicted, but when the bib is moved up and over the patient's 60 face, the adhering element assists in retaining the vestibular device 64 in the desired position over the patient's 60 face. The embodiment shown also comprises a collar 74 that attaches the bib-shaped ALMS 51 to the patient.

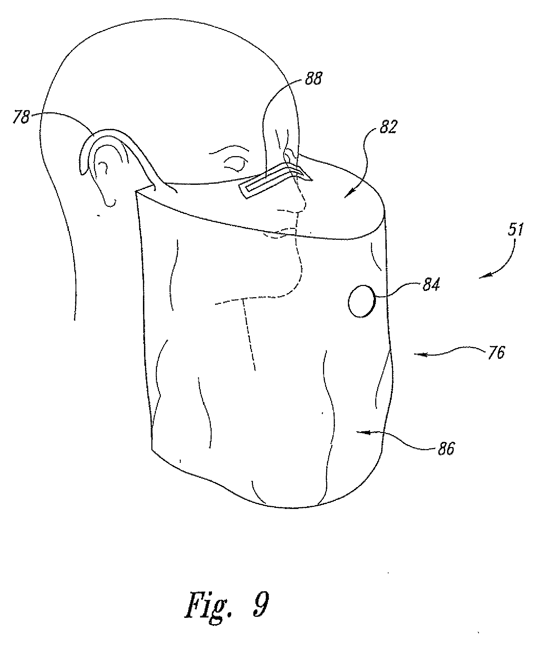

FIG. 9 depicts a vestibular device 76 of an ALMS 51 comprising a brow piece 82, a port 84 and a drape 86. The vestibular device 76 comprises ear pieces 78 that attach the vestibular device to the patient and also help to maintain it in a desired position. This device is particularly useful for supine, angled and fully erect examination of a patient. The embodiment also comprises a seal 80 that is typically malleable and assists in maintaining the vestibular device 76 to the patient and/or in a desired location on the patient.

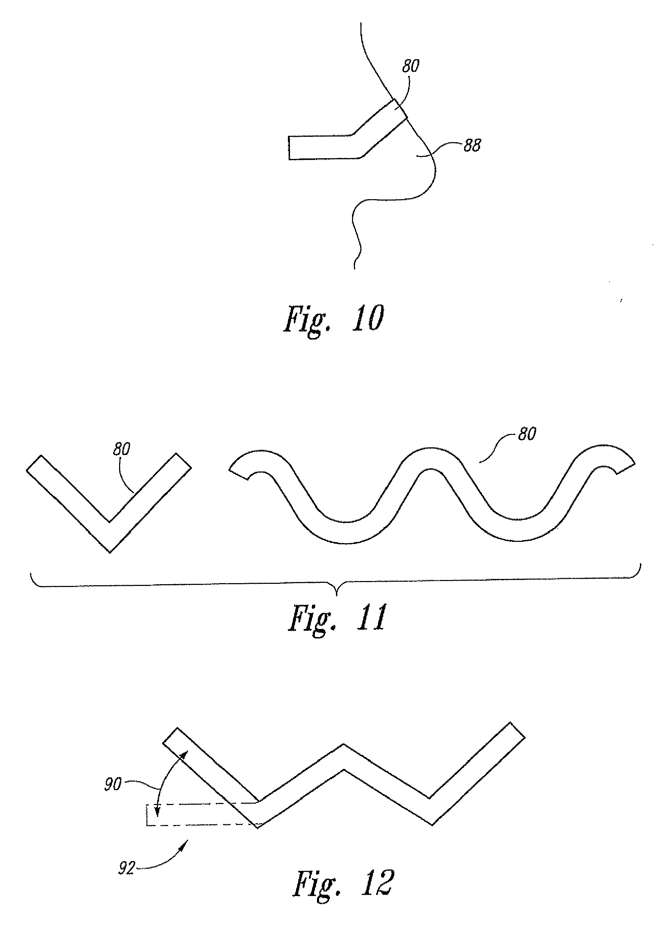

FIGS. 10-12 depict schematically angular and curved embodiments of seals 80 that can be configured to about a nose 88 of a patient. Of course, for other body parts, other configurations would be preferable. As depicted in FIG. 11, the device can be angular or curved, or both. Moreover, although the embodiments depicted use only a single seal mechanism, a plurality of mechanism used either in series or concurrently or otherwise as desired, can be used. As shown in FIG. 12, the seal 80 is typically malleable so that a portion 92 of the seal can be moved about an angle 90 into a desired position. In addition, if desired the seal 80 can be manufactured or otherwise put into a flat shape to ease shipping, storage, etc., and then reconfigured into a non-flat shape at time of use.

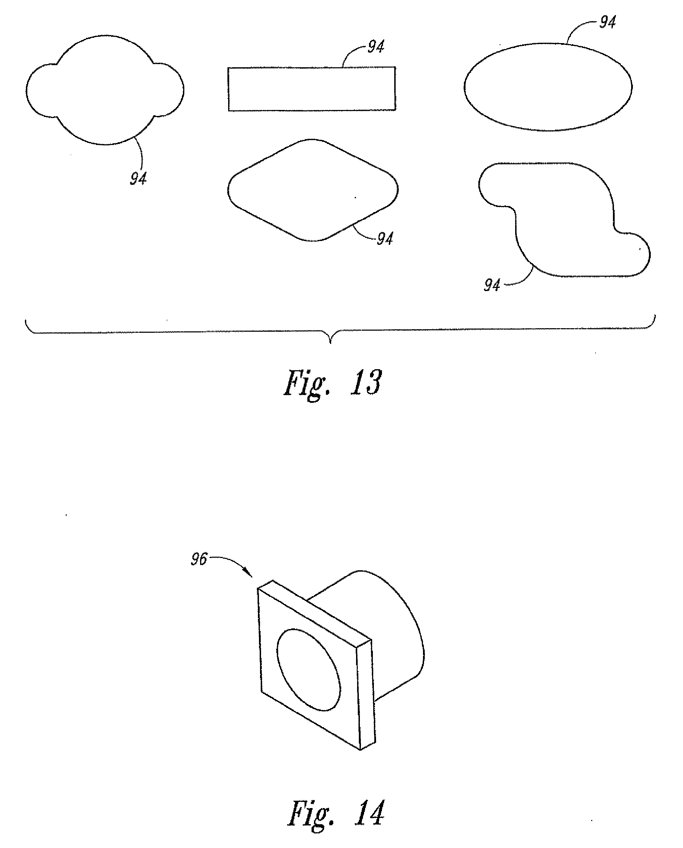

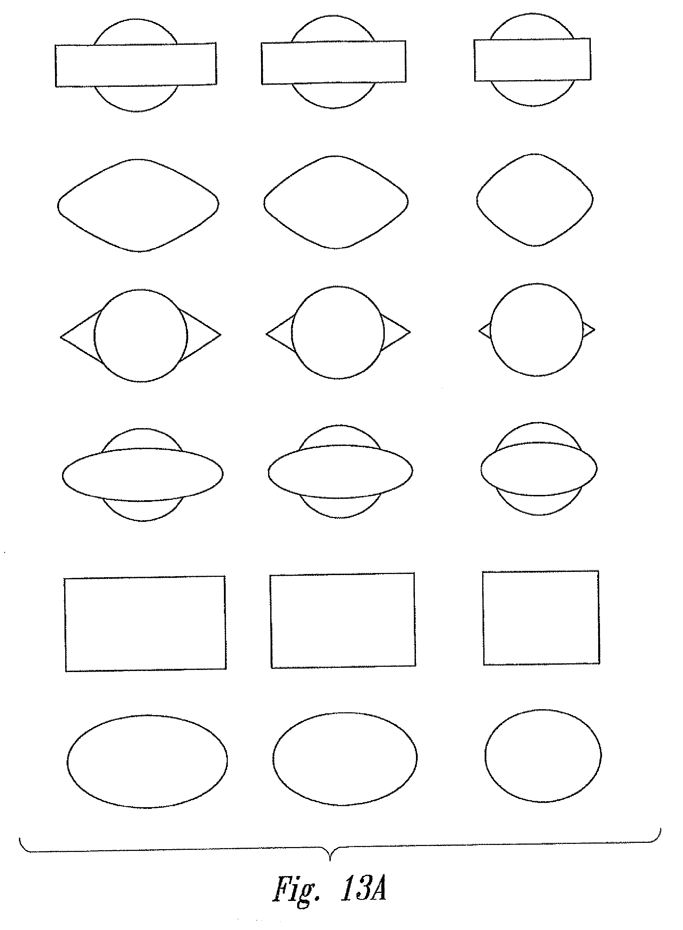

FIGS. 13-13A depict a variety of different shapes that can be used for the port for the viewing scope, the illumination light, the tool or the otherwise as desired. In the embodiments shown, the ports are shaped to be retainable attached to a distal portion, typically the distal tip, of a viewing scope., As can be seen, the ports 94 can be circular but can also be a variety of other shapes such as rectangular, oval, diamond, etc., so that the port can be snapped and locked into place and a lock and key type configuration with the viewing scope. In addition, as depicted in some of the embodiments, the shapes need to be precisely symmetric, which can assist in avoiding undesired attachments of the scope to the port.

FIG. 14 schematically depicts an end piece 96 that can be removable attached to the viewing scope., light source, etc. Preferably, the removable end piece is either disposable or sterilizable so that it can be used repeatedly with multiple patients, or thrown away after a single patient use, both embodiments reducing the likelihood of contamination from one patient to the next.

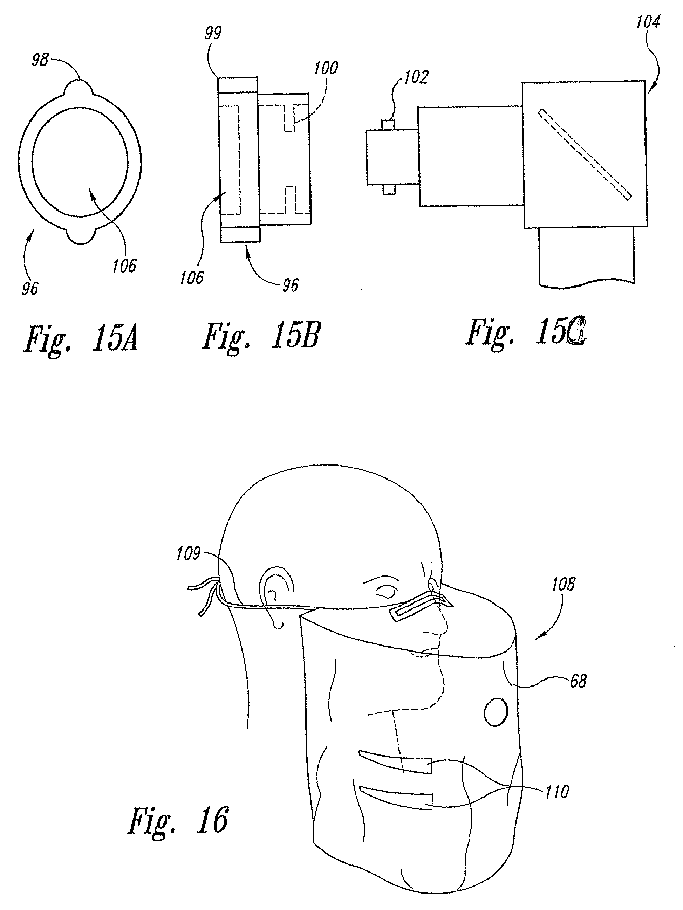

Figure 15 A-C depict one embodiment of a connecting device 97 suitable to connect a viewing scope or light source or other desired instrument 104 to a port in an ALMS. In the embodiment depicted, the distal end 99 of connecting device 97 comprises a port key 98 shape to provide a lockable connection to the port. As demonstrated in other figures herein, a variety of other shapes would also be suitable. The removable end piece 96 also comprises a receiving group 100 configured to receive a nipple or threads or other suitable attachment mechanism of the viewing scope 104. The removable piece 96 can be constructed in either a disposable manner or in a reusable manner. Particularly if the end piece is constructed for reuse, then it is typically preferred that the end piece be suitable for sterilization techniques such as autoclaving, sterilizing washes, etc. As depicted, viewing scope 104 comprises nipples 102 at a distal area of the viewing scope 104 to connect with the removable end piece 96.

Figure 16 depicts schematically further embodiment of a vestibular device 108 when the vestibular device 108 comprises battens 110. Such battens are substantially rigid devices, typically made of plastic, aluminum or other desired material that are sewn in or otherwise attached or adhered to the drape or shroud 68. In the embodiment shown, the battens are located at a mid-point of the vestibular device 108 and serve to retain the vestibular device a spaced distance from the head and neck of the patient. As is also depicted in the embodiment in Figure 16, the vestibular device is retained to the head by a head strap 109 that substantially encircles the head.

Returning to Figures 15 A-C, the removable end piece 96, in the embodiment shown, further comprises a window 106. The window is particularly advantageous for embodiments of the present innovations directed to observations of the oral cavity since a patient's breath can expel contaminating particles that could affect the viewing scope itself or other mechanisms. If desired, whether for the oral cavity or for other body structure, the device can be created without a window. In certain embodiments, the window can function solely as a transparent barrier reducing the likelihood of contamination or other transfer of material from one side of the vestibule to the other, and/or the window can provide a filtering function including either an illumination light or a detection light. In certain embodiments, the window is selected to be substantially non-fluorescing, non-reflective and otherwise substantially inert to the viewing, inspection, tissue excitation, further therapy, etc. functions that can be implemented using the systems and devices herein.

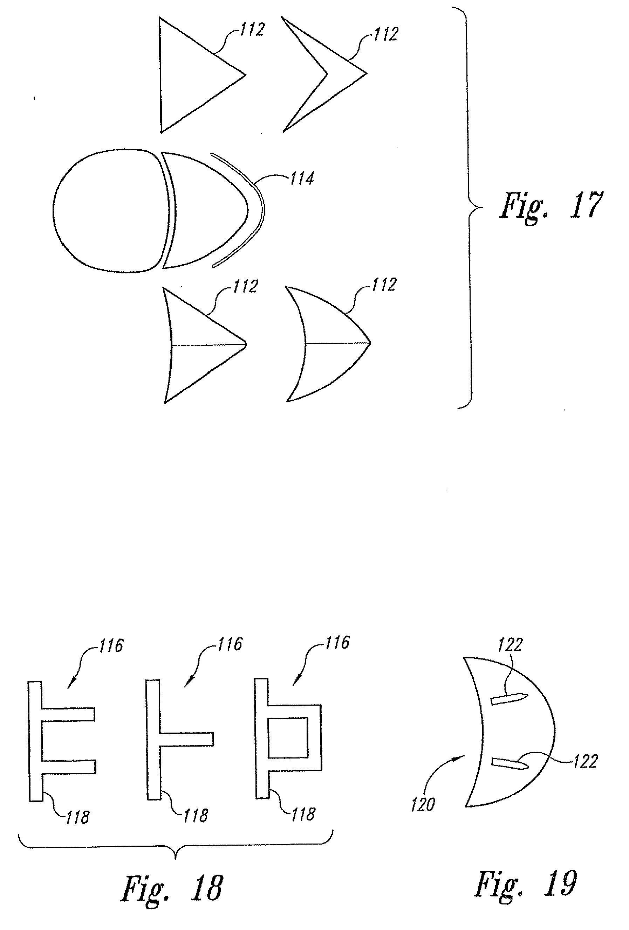

Figure 17 schematically depicts a top view of a plurality of brow pieces 112 that can be used with a drape 114 of a vestibular device to create space within the vestibular device between the vestibular device and the patient. The brow pieces can be single, unitary construction, of two-piece construction, or otherwise as desired.

Figure 18 depicts a top plan view of a plurality of different embodiment of space-making members suitable for use in the brow or other location within the vestibular device of an ALMS according to the discussion herein. As can be seen, legs 118 of the device can support a brow member 116 as shown in this side view. The brow members 116 and legs 118, as with a variety of any of the other structures discussed here, can be valuable and deformable to adapt to various different shape configurations at the desire of the user, for example to better conform or adapt to the features of a given patient's face or other body parts.

Figure 19 depicts another embodiment of a brow piece 120 where the brow comprises of battens 122 to provide the space-making structure that assists in keeping the brow in a desired location.

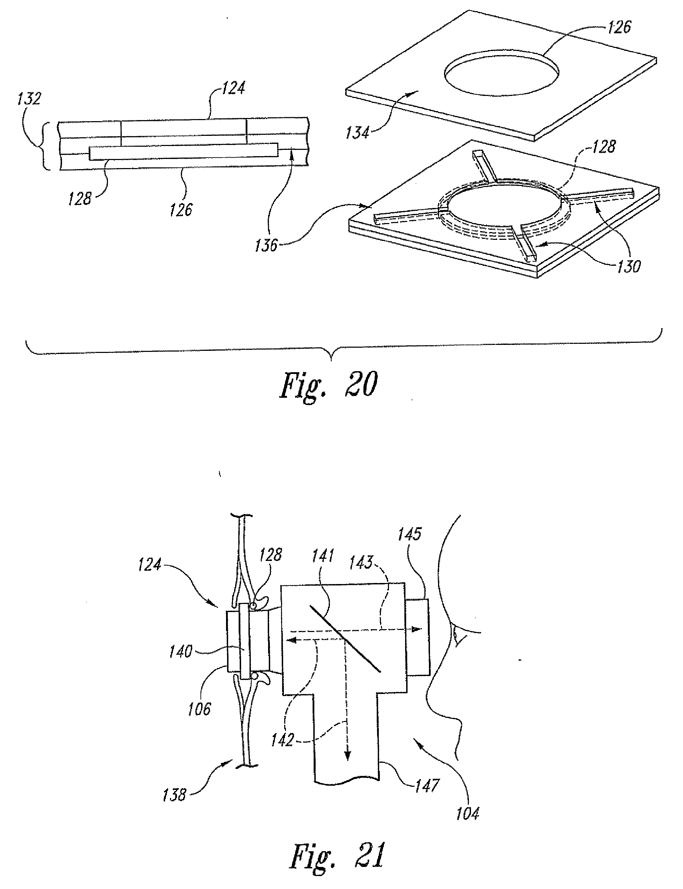

Figure 20 depicts another embodiment for creating a detachable port in a shroud as discussed elsewhere herein. In the embodiment to Figure 20, a port assembly 124 comprises a port 126 defined by an O-ring 128 sandwiched between at least two layers of shroud material 132. In certain embodiments, the different sheets of material 134, 136 are made of a material that weigh less than about 2 ounces per square yard, although material either lighter or heavier can also be used as desired. In a desired embodiment, the port 126 is about one-inch in diameter, although any desired port size, from about 1mm to 1 centimeter (cm) to 3 cm to 5 cm or more can also be provided as desired. If desired, at least one of the sheets of fabric can comprise slits 130 configured to allow entry of a tube, such as a distal end of a viewing scope into the port. The layers of material 134, 136 can be attached to each other via any desired mechanism such as sewing, adhesives such as those from adhesiveresearch.com, Glenrock, PA, including, for example, Removable Adhesive Systems, AR c/o 8561, AS-124M removable adhesive. Further, provided that at least one of the layers of the shroud is adequately opaque, the other layer can be clear or transparent, for example, one of the layers can be configured, as liner comprises 2 mil siliconized clear polyester.

Figure 21 depicts a viewing scope 104 having a light path 142 that transmits light to/from an originating light source and the patient or the patient and a sensor as well as, typically, a direct viewing light path 143 that operatively transmits light from the target tissue to an eye of the user such as a dentist or doctor, which user can see the tissue through eye piece ocular 145. As can be seen, the port assembly 124 in the veil or shroud 138 is preferably releasably, engaged with a lip 140 or other attachment mechanism of the viewing scope 104, which viewing scope as noted, comprises an ocular eye piece 145, as depicted a dichroic mirror 141 and a handle 147. Typically, the direct viewing end of the scope will comprise one or more filters in either or both of the illumination light path and detection light path.

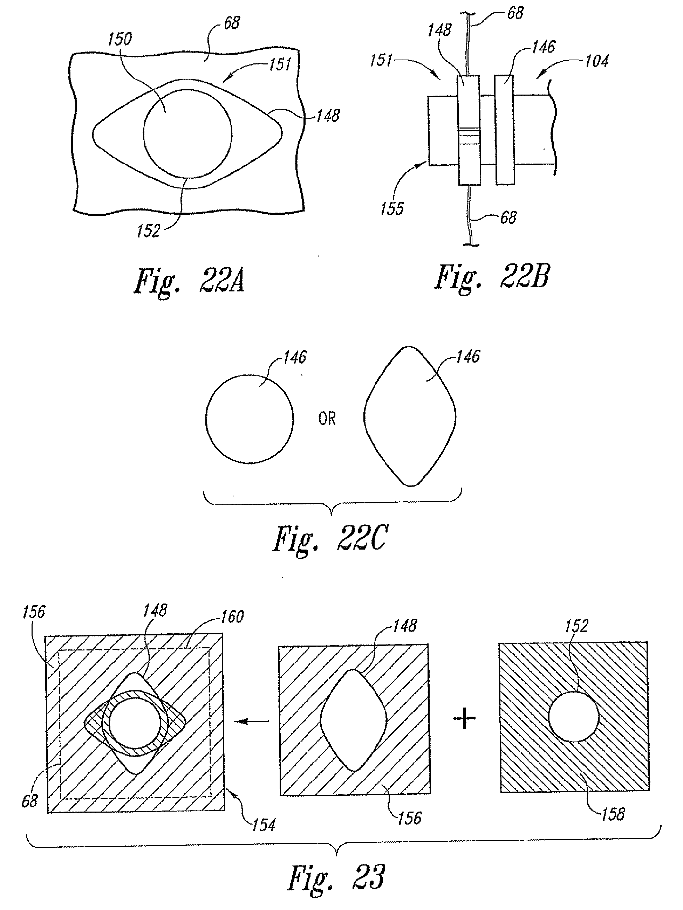

Figures 22 A-C depict a keyway and tube port 151 in a shroud 68 along with a corresponding key and distal end of a viewing scope 104. Briefly, shroud 68 is depicted in cutaway form and comprises a keyway and tube port 151 comprising a window 150 disposed within a port 152, which in turn is disposed within a keyway 148. As depicted, the keyway 148 is diamond-shaped, but any desired shape, including a symmetrical shape can also be used. Figure 22 B takes a side-view of the keyway and tube port 151 partially slid on to a distal end 155 of a viewing scope 104. The viewing scope 104 comprises a restraining ring 146 that is complimentary to the shape of the keyway 148. Exemplary shapes for the restraining ring 146 are shown in Figure 22C. Moreover, the restraining ring 146 can comprise both shapes, for example a circular shape distally located on a diamond-shape such that the circular shape will extend into and through port 152 while diamond-shape can releasably engage corresponding diamond-shape keyway 148.

Figure 23 depicts one embodiment before the manufacturer of a shroud 68 such as depicted in Figure 22A. In this embodiment, a first piece of material 156 having a diamond-shaped keyway 148 is combined with a second layer of material 158 having a substantially circular port 152. Typically, the second piece of material 158 is the side of the drape 68 facing the patient. The two pieces of material can be combined in any desired manner, for example using stitches 160, or a grueling, interference fit or other desired attachment.

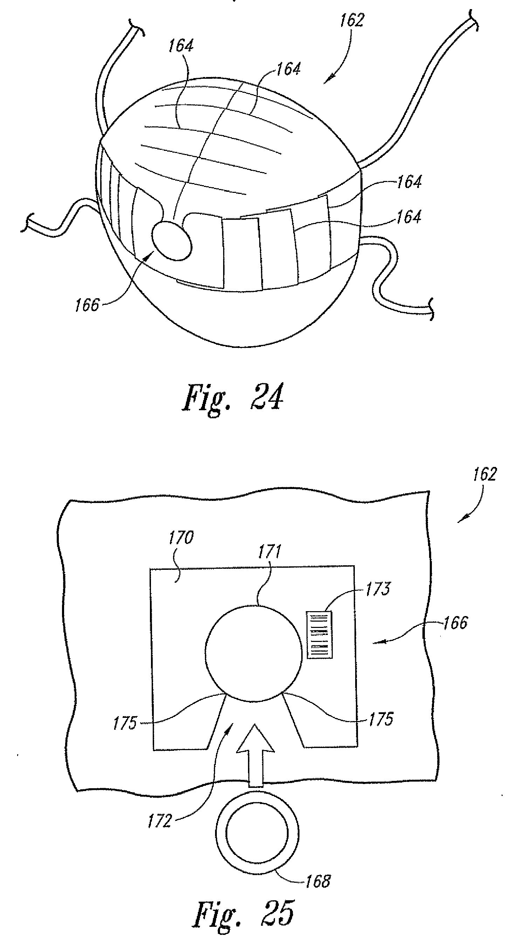

Figure 24 depicts schematically a vestibular device formed by a mask 162 comprising a top-entry docking piece 166 and a plurality of stiffeners 164. The optional stiffeners can be made of aluminum, plastic, other lightweight material whatever material is decided to help provide shape to the mask 162. If desired, the mask can be created without any added stiffeners. Generally speaking, the more the number of stiffeners, and/or the greater the stiffness of the stiffeners the more the mask will rigidly maintain its shape despite exterior pressure or other influence. On the other hand, reducing or eliminating these stiffeners or the stiffness thereof provides a more pliable mask. Figure 25 provides a close-up view of the docking piece 166 depicted in Figure 24. Briefly, docking piece 166 has a body 170 and, in the embodiment shown, a barcode 173. A port 171 is sized to close-fit with a nozzle 168. In the embodiment shown, such close-fit is effected via two projections 175 configured to receivably accept nozzle 168 and to retain nozzle 168 at the desired location in front of port 171. In Figure 25, the passage 172 in body 170 has been inverted such that the device in Figure 25 is a bottom-feed device, as opposed to the top-feed device in Figure 24. Of course side-feed and other-feed devices can also be used.

Identifying symbol 173, which in the embodiment shown is a barcode, but could also be a 3-D barcode, other scanable identifying indicator, and mechanical-fit identifying or other identifying system, such as a right source or viewing scope - specific mechanical fit between the vestibular device or a removable end piece can be particularly advantageous for a variety of reasons. For example, the end pieces and/or vestibular devices, and/or viewing scopes, etc. can be provided with such identifying devices to assure that particular filter combinations are used correctly, or that other particularly embodiments are used correctly, for example assuring that an AMLS system wherein the anti-cross-contamination window is located in the shroud itself is used with a removable end piece (if any) and a viewing scope that do not have such a window so that unnecessary optical interference is not introduced in the system, while also assuring such a vestibular device having a window is used since otherwise an open passage would be created between the patient and the relatively more expensive optics and mechanisms of the direct viewing device, etc. In addition, such can be used to confirm that manufacturer's filters are used properly with carefully selected other filters such that a system suited for use for fluorescent investigation is appropriately provided in all parts of the system, and is not mixed with a system configured specifically for IR investigation. The identification symbol 173 can be located at any desired location, for example, as depicted, on the face of the docking port, elsewhere on the vestibular device, within the ring or other docking portion of the vestibular device such that the vestibular device will only fit a particular desired examining device, tool, etc., or on the tool or examining device itself, or on a removable end piece or on multiple ones of such structures, or as otherwise desired.

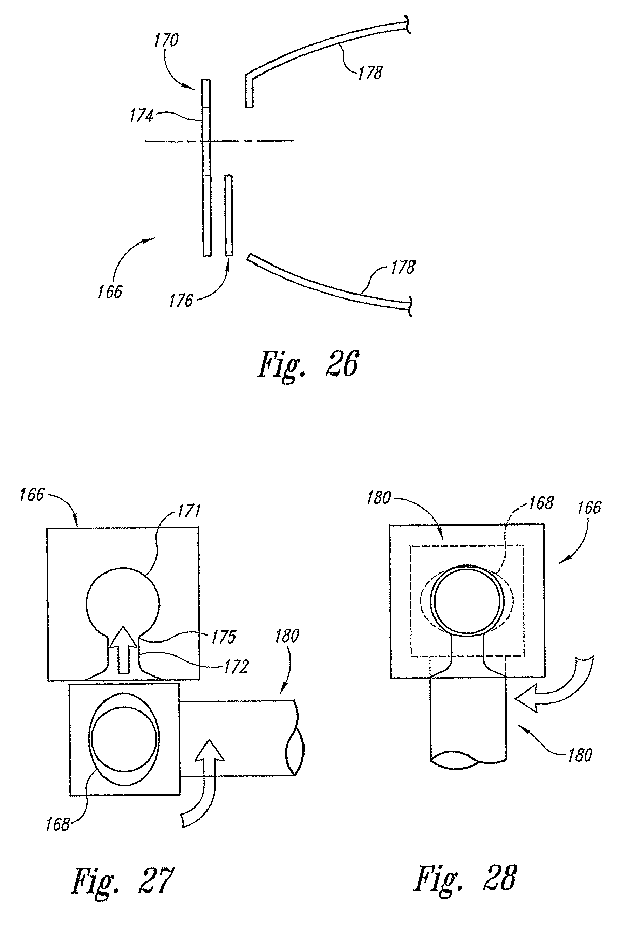

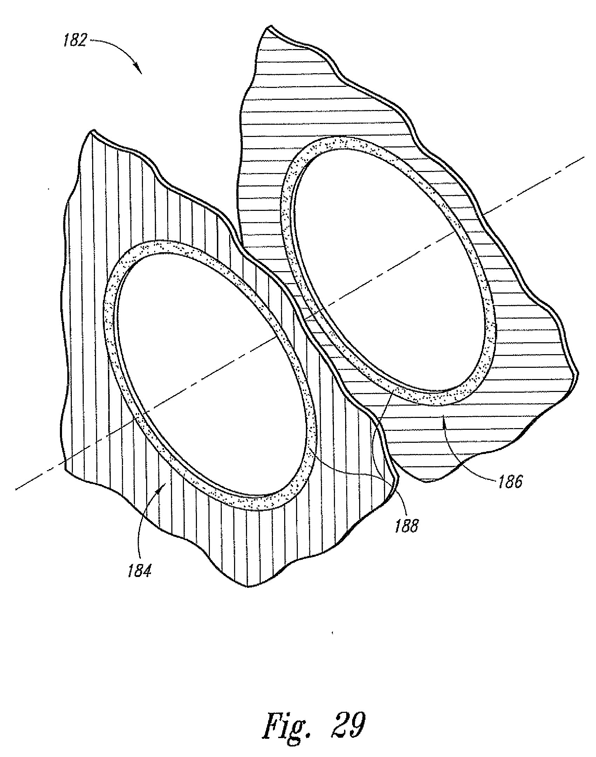

Figure 26 depicts a docking piece 166 comprising a body 170 in which can be made of Mylar, cardboard or any other desired material, a key hole 174 and backing 176, all of which is attached to a veil 178. Figures 27 and 28 demonstrate the docking of a light source 180 (or viewing scope, etc.) when the viewing scope is initially turned as depicted in Figure 27 such that an oblong nozzle 168 can be pushed through passage 172 and the entire light source 180 is given a 90° twist clockwise to engage the oblong nozzle 168 with the projections 175 of body 170 such that the nozzle cannot be removed until the light source 180 is twisted back to the original position and pulled out. Figure 29 depicts yet another embodiment of such a docking system wherein an outer fabric 184 of the port 182 defines a substantially oval hole while an inner fabric 186 defines a substantially circular hole. Each of the passageways is defined by a seam or lip 188 that can be, if desired, configured to provide stiffness.

Figures 27-28 depict a front plan view of a twist-and-lock configuration of a slide-in port.

Figure 29 depicts close up side view port and a vestibular device comprising multiple layers of fabric and having differently shaped passages to form an interference-fitting port for a viewing scope.

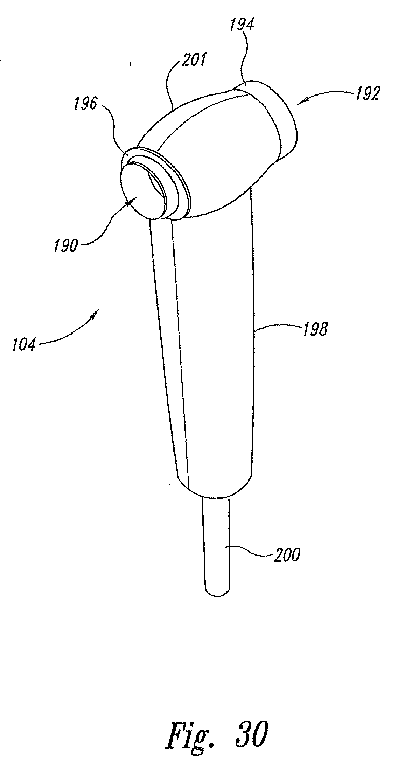

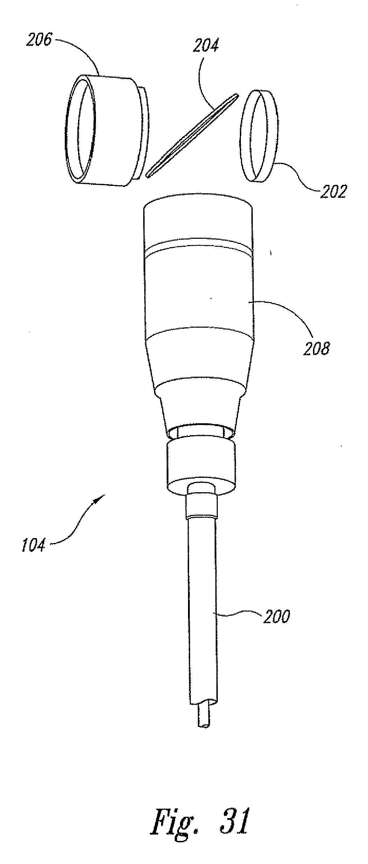

FIG. 30 depicts an elevated perspective view of a viewing scope 104 having a distal end 190 and a proximal end 192. Proximal end 192 comprises an eye cup 194 that serves as an ocular eye piece and provides a user with a comfortable place to rest their eye while simultaneously blocking most ambient light from interfering with the interrogation by the user. Distal end 190 comprises a connector region, which as shown comprises a substantially circular flange and lip; other configurations are also suitable. Viewing device 104 additionally comprises a handle 198 which in the embodiment shown is attached to a conduit 200 that leads to any externally located items such as a power source, light source, imaging equipment such as CCD, CID, CMOS or other digital or analog cameras, imaging devices, etc., as well as spectral-analysis devices such as spectrometers, spectroradiometers, spectrographs, etc. In the embodiment shown, the head 201 of the viewing scope 104 is substantially foreshortened relative to the handle. The configurations are also suitable, but providing a substantially short head 201 has certain advantages in certain embodiments because the relatively short length of the head allows the doctor, dentist or other user to get as close as possible to the target tissue and further may ease the ability to look in a variety of directions in closed spaces, such as during colposcopic or oral examinations. Further, although in the embodiment shown the viewing scope is connected to external power or other devices, if desired the entire system can be maintained in a single, fully portable device that is not attached to anything. Figure 31 depicts a partial element side view of a viewing scope 104.

FIG. 31 depicts a partial element side view of a viewing scope 104 wherein certain elements of the head and handle have been removed to allow viewing of internal elements. In the embodiment shown, an objection/collection tube 206 is disposed towards the distal portion of the viewing scope 104 with a wavelength selective optical element such as a beam splitter 204 substantially essentially disposed in the head of the viewing scope 104 and a filter 202 is disposed at the proximal portion of the head. Handle components 208 maintain within the handle are also shown.

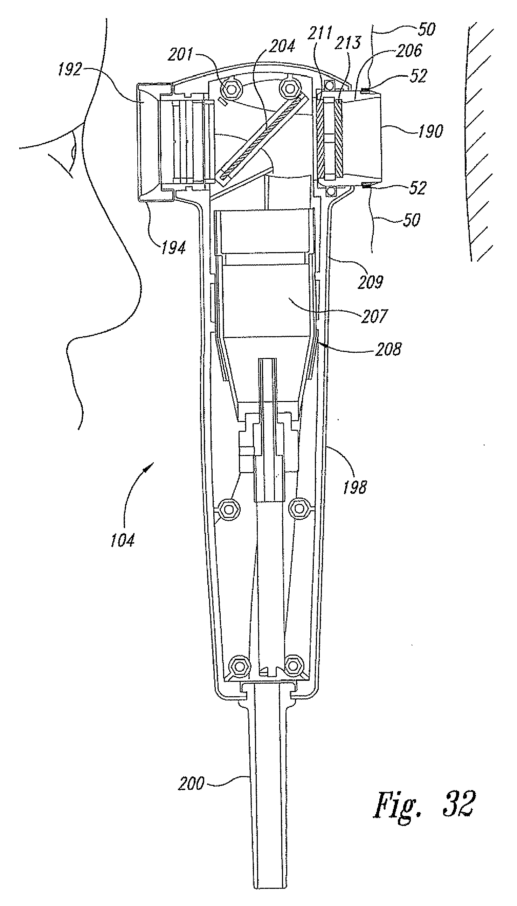

FIG. 32 depicts a cut-away side view of a viewing scope 144 comprising a distal end 190and a proximal end 192. The device further comprises an eye cup 194, and a beam splitter 204. The beam splitter 204 permits light to be projected via conduit 200 into the internal space 207 of the viewing scope 104. The light is then projected upwardly through an optical element 209 into beam splitter 204 which reflects the light through the passage in distal end 190. As noted elsewhere, the light can comprise excitation light suitable for inducing fluorescence, whit light, infrared light, or other types of light as desired by the user. Further, if desired, the light can be provided such that it can be selectively tuned or varied by the user and/or switched between different viewing or illumination modes. Further, the light can be used for reflection-based illumination, fluorescence-excitation based illumination, for therapeutic purposes, for diagnostic purposes, or otherwise as desired. In the embodiments shown, a colposcopic region of a patient is examined by the user. The light returning from the patient is collected by the projection/collection tube, 206. As can be seen, the projection/collection tube 206 is releaseably engaged at port 52 with vestibular device 50. The light emanating from the patient is transmitted through the selective optical element 204 and on to the eye of the user. If desired, emanation light from the patient can also be transmitted by beam splitter 204 back down through the handle to one or more of a camera, analytical device such as a spectrograph, or other analytical elements. Further, if desired, the viewing scope 104 can comprise one or more filters, for example filters 211 and 213. If desired, the filters can filter solely illumination light or, solely emanation light, in which case in the embodiment shown the filters would typically be located either before or after the beam splitter, or, if desired, the filters can function to filter both illumination and emanation light in which case the filters would typically be laced n front of the beam splitter, as is shown in the present embodiment.

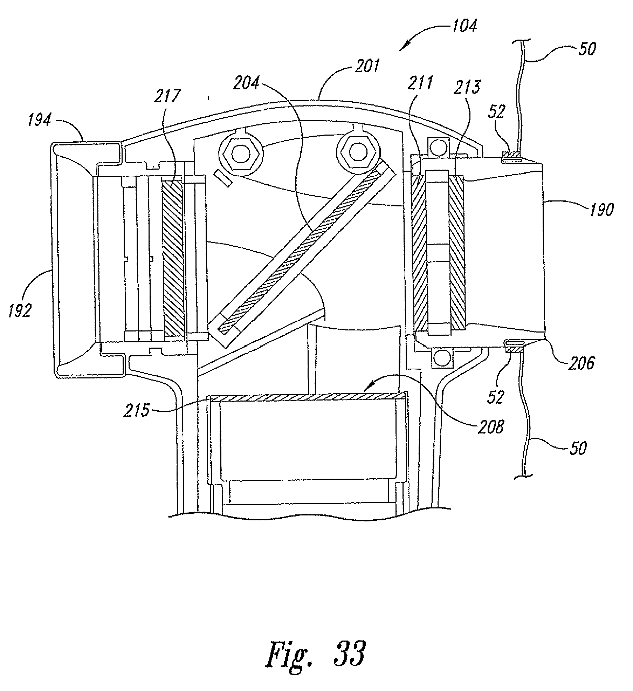

FIG. 33 depicts a cut-away side view of the head of the viewing scope 104 depicted in FIG. 32. In addition, a third filter 215 and a fourth filter 217 disposed before and after the beam splitter 204 have been identified.

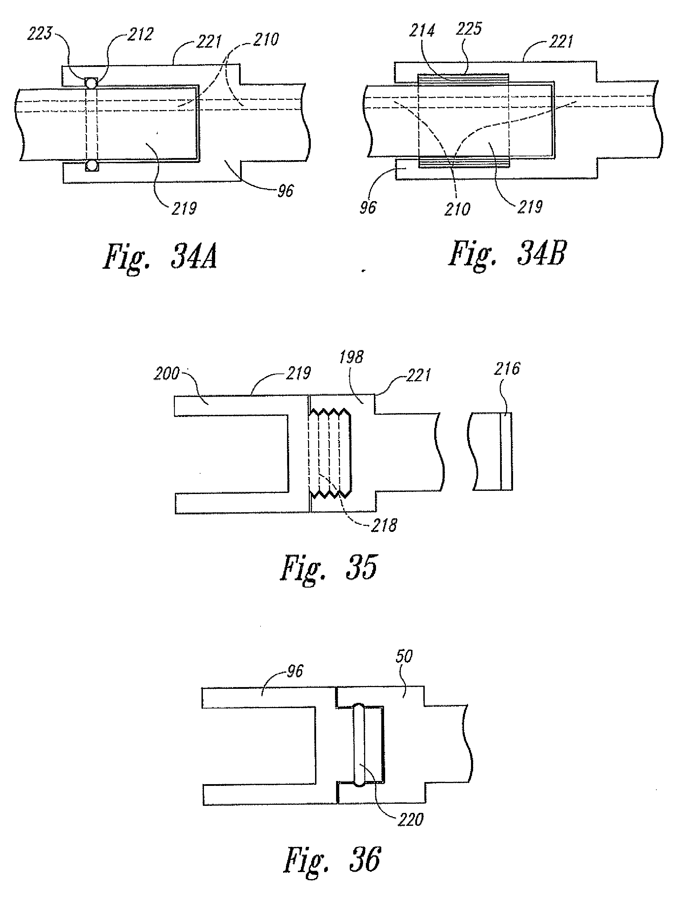

FIGS. 34-43 depict schematic side, cut-away views of a variety of connection mechanisms to connect eh various devices in the system.

FIG. 34 depicts one exemplary connection assembly suitable for attaching the viewing scope 104 to the conduit 200, or for attaching a distal removable end piece. FIG. 34A and 34B both comprise a light path 210, a male end 219 and a female, receiving end, 221. In FIG. 34A, the male end 219 is retained in operable connection with the female end 221 vi a compressible O ring 212 that fits within a receiving notch 223. The ends can be connected and separated merely by pushing them together and pulling them apart. Similarly, in FIGS. 34B, male end 219 is retained in female end 221 via a wide compliant material 214 that snuggle fits within a complimentary wide notch 225. The O ring, compliant material, etc., can be formed with any suitable material such as plastic, sponge, rubber, etc. As can be seen, in the desired embodiment, the optical pathway is aligned for operable connection.

FIG. 35 depicts an alternate connector wherein the male end 219 of a conduit 200 is attached to a female end 221 of a handle 198. The connection is effected via screw threads 218. If desired, the device can further comprise a window 216 which is typically non-fluorescent, transparent, and non-reflective.

FIG. 36 depicts a ridge and groove fitting that functions similarly to the embodiments discussed in the preceding figures. In this embodiment, a snap over ridge 220 is provided to enhance the retention of the two ends to each other.

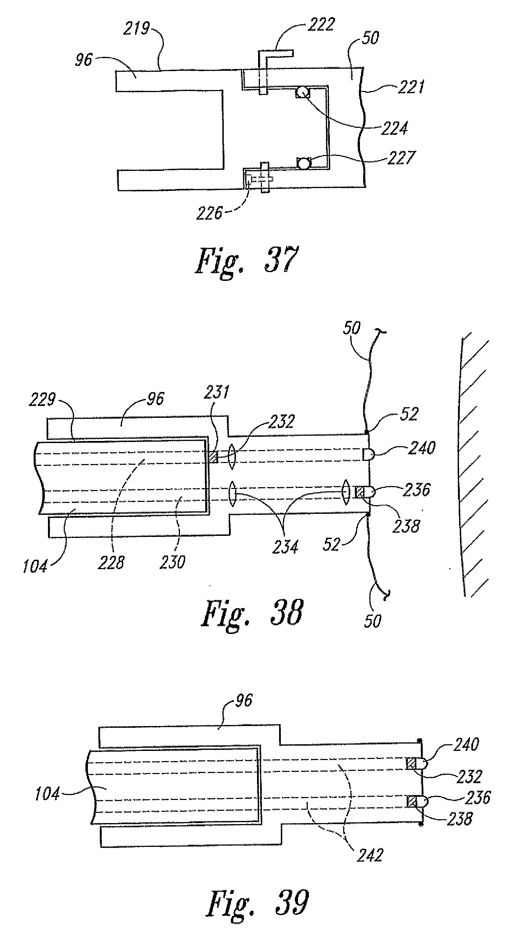

FIG. 37 depicts another quick disconnect fitting wherein a latch lever 22 works in cooperation with a spring load pin 226 to releasably and securely hold the male and female ends together. In the embodiment shown, a O ring 224 maintained on the female end 221 interacts with a notch 227 in the male end 219. The spring-loaded pin 226 and the latch lever 227 cooperatively interact to lock the two ends together and therefore provide a mechanism for more secure holding that would be less likely to come apart if inadvertent substantial pressure is applied.

FIG. 38 depicts an embodiment of the removable end piece 96 directed to the illumination and collection optics. Briefly, removable end piece 56 slides onto a suitable projection 229 of a viewing scope 104. Illumination pathway 111, which can be a hollow tube, a fiber optic cable, a fiber optic bundle, a liquid light guide, or any other desired light guide transmits illumination light from the viewing scope 104 through illumination pathway 228 then past a band pass filter 232 a collimating lens 234 and an output lens 240. The light is then transmitted to the arm of the patient, where fluorescent, reflection, Raman signals, or other emanating light from the arm of the patient is then transmitted to collection lens 236, transmitted past a high pass filter 238 and collimation lenses 234 and into receiving light path 230. In the embodiment shown , filter 234 is a band pass filter that transmits substantially only excitation light suitable for inducing fluorescence in fluorophores (either auto fluorophores or fluorophores provided via drug, fluorescing agent, etc.) in the target while high-pass filter 238 blocks substantially all of the excitation light and transmits substantially only the fluorescent light emanating from the patient.

In the embodiment in FIG. 38, band pass filter 232 projects from the projection 229 into a complimentary receptacle 221 in adapter 96, thereby providing a suitable identifying and device management system such that the illumination light path and the collection light path are not confused during use. FIG. 39 depicts a similar embodiment focusing on the optics.

In FIG. 39, the optics include fiber bundles 232, of course other suitable light guides can also be used, as well as an output lens 240, a band pass filter 232, a collection lens 236 and a high pass filter 238. In this embodiment, the device does not have the protecting band pass filter 232 and the corresponding receptacle 231 depicted in FIG. 38, so optical connection within the various elements of the device would typically be determined by the user continuing operable signals.

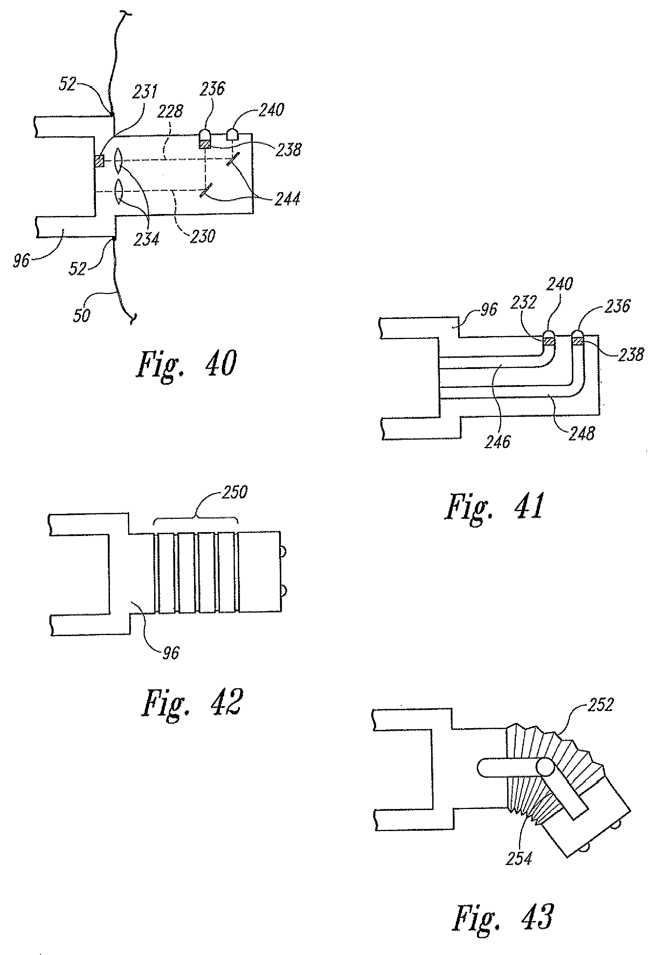

FIG. 40 provides a cut-away view of an adapter 96 comprising lenses and mirrors to provide side viewing capabilities. Illumination light path 228 proceeds through receptacle 231 past collimating lenses 234 and onto an angled mirror 244 that directs light out through output lens 240 that is located on the side of the adapter 96. Collection light path 230 collects light through collection lens 236 which is transmitted past angled mirror 234 and then proximally through collimating lens 234 to the viewing scope. FIG. 41 depicts an alternative side-viewing adapter. In FIG. 41, adapter 96 comprises light guides 246, 248 that bend the light from the viewing scope (not shown) and then project and collect the light through respective collecting optics 246 and illumination optics 248.

FIGS. 42 and 43 depict side views of two flexible adapters. In FIG. 42, a corrugated goose neck 250 is provided, which gooseneck can be bent and twisted as desired by the user, within the limits of the materials selected for construction of the device. FIG. 43 provides a flexible rubber hosing 252 that can be freely bent and twisted, and a linkage 254 that serves to stiffen the adapter as desired and to retain it in position.

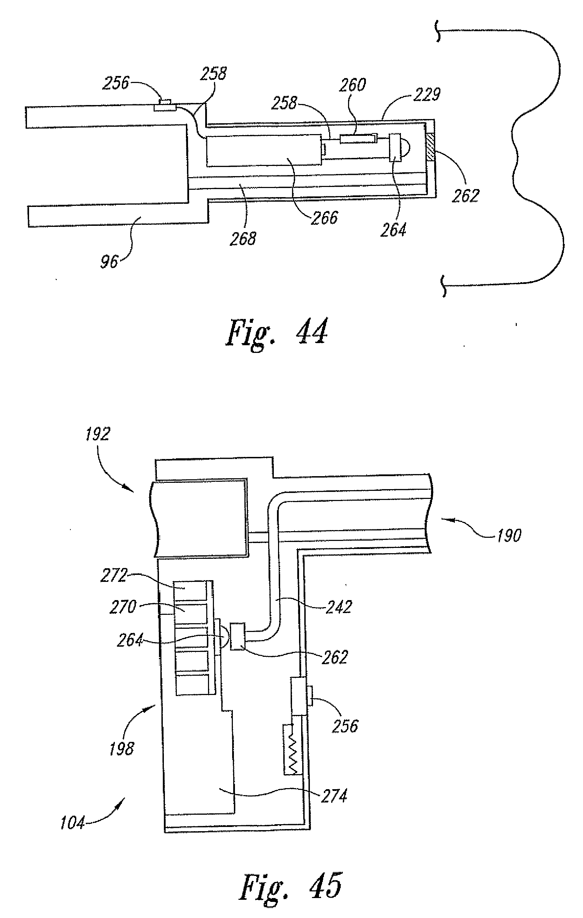

FIG. 44 depicts a cut-away side view of an adapter comprising both a power source and light source. Briefly, projection 229 of adapter 96 contains a battery 266 operably connected to a light source such as an LED 264. As depicted, the wiring 258 passes through a current limit register 260 so that the intensity, and possibly color, depending on configuration, emitted by the light source 264 can be variable controlled. Battery 266 and light source 264 are activated and controlled via a switch 256 light emitted from light source 264 is transmitted out of the adapter via filter 262 to the patient, and then return light is collected via collection light path 268 similar to those described elsewhere herein or otherwise configured as desired. In the embodiment shown, the lights projected from the adapter to an OS application for cervical examination and the return light, likewise, returns from the OS. In certain embodiments, the side-viewing devices discussed herein can be particularly advantageous for such examinations.

FIG. 45 depicts a cut-away view of a viewing scope 104 having a proximal end 102 and a distal end 90. The handle, power source, optics, etc., shown in FIG. 45 can be provides either in a hand held assembly that forms the actual viewing scope itself, or that is adapted to be attached to the viewing scope, typically at the distal end.

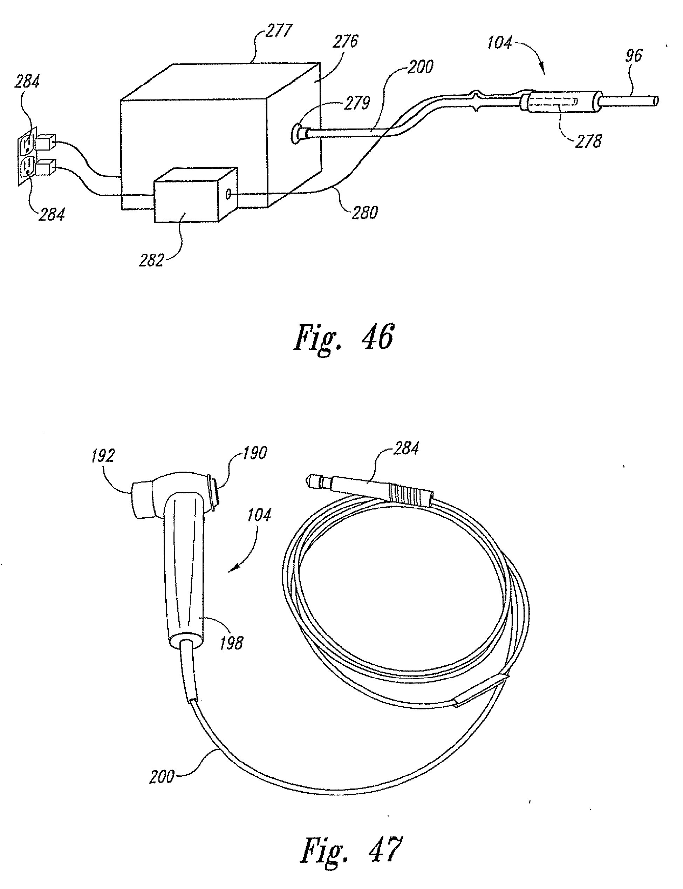

Handle 198 contains a plurality of elements to provide power and light, including a power register battery 274, a fan 272 for cooling, and a heat sink 270, that also assists in cooling. The power register battery 274 supplies power to alight source 264 which emits illumination light into a filter 262 which in turn provides light into an illumination light guide 242 for transmission to the distal end 190. The power, light source, etc., is controlled via a switch 256, which, as depicted, can advantageously be placed on the proximal side of the handle similar to a trigger, but can also be placed in any other desired location. FIG. 46 depicts a perspective view of a schematic figure of an ALMS. In this embodiment, a housing 277 contains the light source, power source, camera and other desired analytical or imaging elements, if any. Proximal side, 276 contains a port 279 out of which extends conduit including light guides, electrical wiring, and other desired elements. The conduit 200 transmits desired signals, light, etc., to viewing scope 104 which comprises a light want 278 and an adapter 96. In the embodiment shown, a power supply 282 is located abetting the housing 277 and has a power cable extending from the power guide to the viewing scope 104. The light source and power source are each plugged into wall outlets 284.

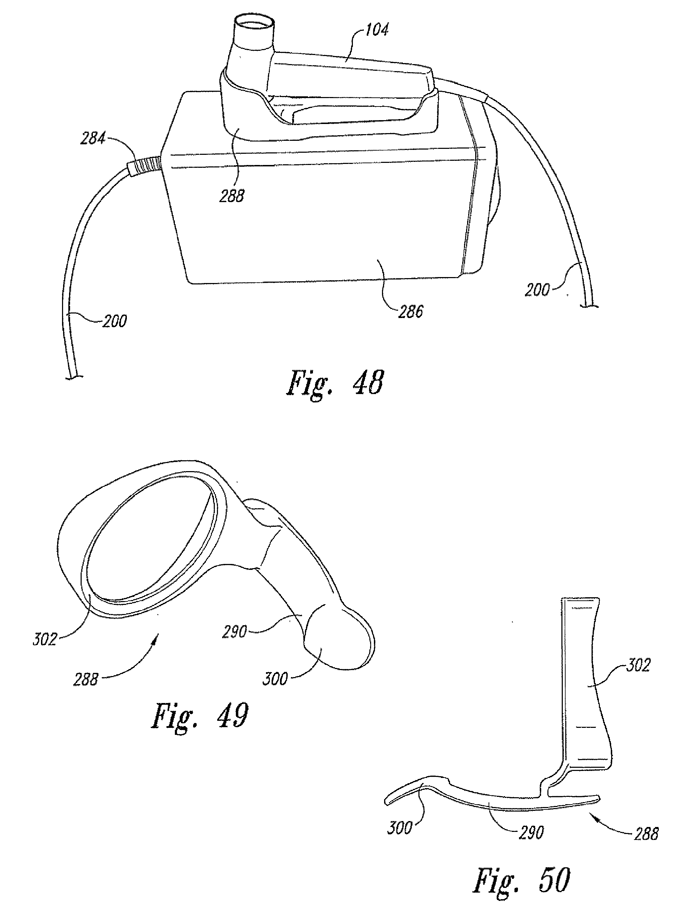

FIG. 47 depicts a viewing scope 104 having a distal end 190 and a proximal end 192 and a conduit 200 leading from the base of the handle 198. As can e seen, the conduit is fairly lengthy, (typically about 4-15 feet in length) and ends in a plug in adapter 284 configured to releasable plug into an external power and light source. Figure 48 depicts the viewing scope and conduit of FIG. 47 attached to the external power and light source.

FIG. 48 depicts the viewing scope 104 connected via conduit 200 and plug in adapter 284 to an external power and light source 286. External power and light source 286 compnses a norder 288 complimentary in shape to the viewing scope 104 and sized to retain the viewing scope in a desired position on the holder.

FIG. 49 depicts a tool 288 comprising an extension 290 having a ridge 300. Configured to attach to the distal end of a viewing scope. FIG. 49 depicts an elevated perspective view of a tool 288 suitable for attachment via a port ring 302. The port ring slips, slides, clicks, or otherwise attaches at the port ring 302 to the viewing scope, typically at a distal end. The tool 288 comprises an extension 290 that is configured to keep undesired issue out of the viewing path. Accordingly, the extension 290 can be configured as a tongue compressor, a vaginal side wall compressor, or otherwise as desired. The tool 288 can be configured to rotate once it is attached to the viewing scope, or it can be configured to attach and remain in a single position or in a movable position wherein friction or other force detains it in a given position to which the user moves it. Further, if desired, the extension 290 can be configured to be extendible and retractable or, as depicted, can have a single length.

FIG. 50 is an elevated side view of the device of FIG. 49.

FIG. 51 depicts an exploded view of a viewing scope 104. Handle 198 is retained to head 201 via bolts 304, which are covered for aesthetic and cleanliness purposes by both covers 306. A side handle 304 s an internal scaffold 308 that maintains various elements in desired positions and also provides additional strength. Typically the scaffold, the handle, the head, etc., are made of plastic or other light weight material but can also be made of metal or other medically acceptable material and are also typically made so that they can be autoclaved, sterilized with liquid sterilizing fluids, or to otherwise treated to maintain the integrity of a medical or dental environment. The viewing scope 104 is depicted in combination with a tool 288. The tool 288 is a two-piece tool in the embodiment shown, comprising a ring 302 that snaps via a bayonet lock 316 connection system to the distal end 190 of the viewing scope 104.

Extension 290 of tool 288 then snaps onto ring 300. Retaining ring 300 contains plurality of retaining ridges 318 that snap onto extension 290 and hold it in place. As can be seen, the extension 290 can be attached and detached and placed abut almost any location on the ring 302.

Scaffold 308 contains a plurality of optical elements such as filters and mirrors which can be seen by their retaining structures. For example, a short pass filter 314 is retained at a structure between the light source and the retaining structure for dichroic mirror 141. In the embodiment depicted, the short pass filter 314 passes substantially only blue light, i.e., light having a wavelength less than about 450-500nm, which is light that is substantially safe for projection onto a human (without the potentially deleterious effects of UV light) while still being of adequate energy to induce fluorescence such as auto fluorescence or fluorescence from provided fluorophores. Other filters having other characteristics can also be provided at this location, and if desired, a replaceable slot or other structure can be provided so that different filters can be inserted into and taken out of a single viewing scope. Alternatively, multiple viewing scopes having different filter combinations can be provided.

Dichroic mirror 141 reflects light from the light source through the distal end 190 of viewing scope 104 and window 303 of tool 288. Returning light from the patient passes back from window 303 dichroic mirror 141. In the embodiment depicted, dichroic mirror 141 functions as a long pass filter having a cut off of about 475-480nm, i.e., it blocks light of shorter wavelengths and transmits light of longer wavelengths. In the embodiment depicted, the dichroic mirror 141 blocks substantially all of the excitation light passed by short pass filter 314. Proximal to dichroic mirror 141 is a retaining structure for a second long pass filter 310 that eliminates substantially all non-desired light passed by dichroic mirror 141. In such an embodiment, essentially all of the undesired light is eliminated for the viewer. If desired, still further filters can be provided, such as notch filter 312 retained at a retaining structure downstream from the second long pass filter 310. In one embodiment, the notch filter passes substantially all light except for light between about 585-595 nm. This particular embodiment is particularly advantageous for detection of oral cancers, cervical cancer, and other cancers in part because elimination of such light helps the viewer to discern between diseased and non diseased tissues. Such a notch filter can also be particularly useful for the detection of other lesions, particularly lesions that involve red-shifted fluorescence signatures pursuant to the presence of the disease or condition.

FIG. 52 depicts a schematic view of a binocular assembly 326 comprising binocular viewers 320 which in the embodiments shown are configured substantially as glasses comprising independent lenses, one for each eye, lenses 330 and earpieces 328. The binocular viewers 320 are adapted to be releasably connected to binocular ports 322 at a junction 323 the adapter 324 then combines the light path for each of the binocular lenses into a single interrogation light path exiting through port 332. In other embodiments, a binocular viewing capabilities of this embodiment can be continued into the actual interrogation area, typically therefore, comprising separate viewing ports, and, if desired, separate illumination zones. This can in some instances be particularly advantageous because different wavelengths of light or different rays of light having other different properties such as different polarization properties, can be provided for examination of different areas or the same area independently by each eye of the user.

Further general discussion.

In certain embodiments, the scope provides a fluorescent excitation light, for example by passing white light past (e.g., via transmission or reflection) a filter that passes substantially only light within the excitation range, for example 400-450 (in certain embodiments, it is preferable to use light substantially only longer than about 400 nm so that UV light, which may itself be dangerous, is not included in such light). Exemplary filters include short pass and band pass filters.

The excitation light then contacts the target. Response light then emanates from the target, typically via fluorescence although reflectance light or other light such as IR light or Raman can be used sometimes, either in addition to or instead of the fluorescence light. The scope device itself then processes the emanation light collected by the scope and transmits it to the viewer (and, if desired, to other device(s), such as a camera, video detection device such as a CCD, CID, CMOS, etc., or a computer or spectrometer or other spectral-measuring device).

The detection light path of the scope typically has at least one long pass filter, or other desirable filter, that substantially blocks the excitation light such that only light of longer wavelengths can be seen. In one embodiment, such blocking is effected by providing a dichroic mirror having a cutoff of about 475-480 nm (*i.e*., it blocks light of shorter wavelengths and transmits light of longer wavelengths), followed by a "green glass" filter that also blocks light of shorter than about 475-480 nm and transmits light longer than such wavelengths.

In certain embodiments, the scope further comprises at least one filter that processes light such that different wavelength bands of the detection light remain visible while other wavelengths band(s) are either substantially eliminated or substantially reduced in power or intensity. For example, downstream from the long pass filter(s) the scope can have a notch filter that passes substantially all light except for light between about 585-595 um. This particular embodiment is particularly advantageous for detection for oral cancers and certain other lesions.

Other suitable notch filter(s) can also be provided, for example to enhance discrimination between oxygenated and deoxygenated blood (i.e., oxyhemoglobin verses deoxyhemoglobin), or to distinguish between activated and inactivated drugs or diagnostic markers. If desired, one or more variable or non-variable ratio wavelength scaling filters, such as discussed in U.S. patent 6,110,106 can also provided, so that the respective ratio of the differing transmitted wavelength bands can be varied in desired embodiments. Additional examples of multiple band pass filter combinations are also discussed in U.S. patent No. 6,021,344. As noted above, both of these references are incorporated herein by reference in their entirety for all of their teachings and disclosures, including their discussions of endoscopes, filters, suitable detectors, and light sources, etc.

If desired, the scope of the present invention can be substantially a hollow body containing the filters, typically placed on a handle for ease of use. In some embodiments, the device can further comprise an extension member configured to attach to the distal end of the scope (or elsewhere as may be desired) which extension member can be used to push interfering tissue or body structures, such as the cheek, tongue, or walls of the vagina, out of the way of the view of the scope so that a better view can be obtained. Examples of some embodiments of such a device, sometimes called a "retractor," are included in the figures herewith In one desired embodiment, the tissue movement device is rotatably attached to the scope so that it can be more easily or effectively moved within the oral cavity or other body cavity under investigation.

The physical composition of the scope can be varied significantly according to the particular desires and needs of the user. For example, as noted above, the scope can be substantially only a hollow casing with required optics that merely transmit light from an external (typically proximally-located) light source to the target tissue, and then return the light from the target tissue to the ocular eye piece of the scope (the ocular eye piece is typically an eye cup, but can also be other suitable ocular devices, such as frosted glass, and can be monocular or binocular as desired). If desired, the scope can alternatively, or additionally, be configured to contain one or more internal light sources, distally located light sources (such as LEDs), and/or proximally located light sources, and one or more fiber optic light guides, fiber optic cables or other such light transmission guides, in addition to, or instead of, the light guide formed by the hollow casing with suitable optical elements discussed herein.

Typically, the scope comprises a power source suitable to provide the light. The power source can be an external power source such as a battery pack connected by a wire, a battery pack maintained in the handle or else within, the scope itself, or a plug or other appropriate linking device to a wall outlet or other power source. The figures show exemplary power/light source combinations. In some embodiments, the housing of the light source includes a retaining structure configured to hold the scope when not in use.

As noted previously, the scope can, if desired, comprise one or more non-human detectors, such as sensors comprising CCDs, CIDs, CMOSs, etc., and/or one or more display devices, such as CRTs, flat panel displays, computer screens, etc. In addition, if desired the scope system can include one or more computers that control, process, and/or interpret the various functions of the scope, including, for example, diagnostic, investigative and/or therapeutic functions.

A "computer" is a device that is capable of controlling a filter, selective light modulator, detector or other elements of the apparatus and methods discussed herein. For example, the controller can control the light communication characteristics of a selective light modulator, control the on/off status of pixels of a pixelated light detector (such as a charge coupled device (CCD) or charge injection device (CID)), and/or compile data from the detector, including using such data to make or reconstruct images, as feedback to control the illumination light, or other elements of the apparatus and methods discussed herein such as diagnosis or treatment. Typically, a computer comprises a central processing unit (CPU) or other logic-implementation device, for example a stand alone computer such as a desk top or laptop computer, a computer with peripherals, a handheld, a local or internet network, etc. Computers are well known and selection of a desirable computer for a particular aspect or feature is within the scope of a skilled person in view of the present disclosure.

In one exemplary embodiment, a system herein comprises a light source, handheld scope, a vestibular device (ALMS), a retractor or (cheek pusher), a light pathway, a light box (light source), a light guide and an ocular at the proximal end of the scope. The light path extends from the illumination light path to the target to the user and within the handheld viewing scope comprises in order a collimator, 430+/-30 nm filter (filter 1), a dichroic filter (filter 2), light to avoid absorber, glass window, mucosal tissue or other target tissue, dichroic filter (filter 2 (the light passes back past the same dichroic filter), 475 long pass filter (filter 3), 590 nm notch filter (filter 4), eyepiece ocular. The filters can be either separate (discrete) or combined (e.g., reflective coatings). The systems can also or instead comprise binocular eyepieces such as loops/filtered glasses or sunglasses/goggles with/without magnification, and a curing light. The curing light can have a multi-wavelength system (e.g., a second head with a separate filter), and the wavelength of the curing light can be controlled by independent filters and/or filter wheels, etc. Some other features that can be included are a light wand, mirror and/or fiber optic, typically collimated, or an LED on the wand, or an intra-oral camera, which can have a sleeve with a filter at the end to provide particularly desired light and thus function as the light wand, and thus as the light source or as an additional light source for fluorescence or other desired response.

The intra-oral camera designs can have multi-wavelength light processing within and outside the camera. The light can be piped through the system or a light source can be incorporated or there can be a separate sleeve (or other suitable light emitter) with its own light. The sleeve could have appropriate wavelength emission/excitation filters. Filter and other optical element position can vary within the pathway provided the desired functions are achieved.

The illumination light and viewing pathways can be combined as in certain of the figures, or separate as in a light source with loupes/eyewear. The pathways can enhance user ability to use the device to have a standard method of viewing and illumination. From the point of view of two people looking into the mouth at the same time, both have different viewing angles of the tissue being illuminated and thereby see different tissue and would possibly then make different diagnoses. A tripod is sometimes used to reduce the variability by standardizing height and angle. The size of the spot of interrogation in some embodiments is sized to compare a full lesion to surrounding normal tissue, which enhances viewing and identifying anatomical landmarks for location.

In some embodiments, intensity is optimized to bathe the tissue with excitation light for diagnosis, to excite the necessary fluorophores to produce a signal. In some embodiments, the wavelength(s) is optimized for recognizing certain types of cancers found in the mouth or other target tissue. The wavelengths/fluorescence enhance ability to recognize a shift in the fluorescent emission spectra to permit differentiation between normal and abnormal for cancerous tissue. For example, dual monitoring of two wavelength bands from about 475-585 and from about 595 and up enhances monitoring of cellular activity for the metabolic co-factors NAD and FAD. NAD and FAD produce fluorescence with peak levels at such wavelengths.

In certain embodiments, it is desirable to get as much power as possible without smearing emission signal too much, to keep the output spectrum narrow to prevent Stokes shift, and to exclude UV light and to avoid illuminating/exciting with light in the emission band (overlapping fluorescence).

In certain embodiments, the systems can further comprise a diffuser to make spot-size more regular, remove hot spots, etc. Also sometimes desirable is a collimator to straighten light out at the filter, and to limit the divergence of the beam with increases in power density, or to use a liquid light guide and not fibers so as to get more efficiency by reducing wasted space between fibers, and achieving better transmission per cost and higher numerical aperture (which contributes to better light collection). In still other embodiments, the systems can further comprise metal halide light sources to enhance power in certain emission ranges, dichroic filters or similar optical elements to enhance overlapping viewing and illumination light paths (can simultaneously direct illumination. light away from the source and emanation light from the tissue). A glass or other transparent window at the front of the scope can keep out the dust.

In certain oral and other embodiments, the scope is used about 4" from device to back of mouth or other target; spacers can be provided if desired. The scopes can be black internally to absorb stray reflected illumination and released fluorescent (unwanted fluorescent feedback) light.

The shape of the scope can be preferably set to be ergonomically comfortable, optimize the excitation and emission pathways. The proximal eyepiece can be set at a length, such that tilting the proximal filter (e.g., a 590 nm notch filter) creates a geometry such that incoming ambient light from behind the practitioner can be reduced and what passes can be reflected into the absorbing internal tube surface. This reduces reflection and prevents the user from seeing themselves. For example, the proximal filter can be tilted with its top closer to the clinician and bottom closer to the dichroic mirror so as to make a reflecting surface that would direct incoming light into the bottom of the optical pathway tube.

Detachable eyepieces can allow an adaptor for cameras to be inserted. In certain embodiments, the camera attaches as close to the dichroic as possible, which reduces the optical pathway inside the Scope for the camera, which in turn reduces the chance/tendency of the camera to autofocus on the inside of the VELScope. This can be also important as fluorescence produced can be low intensity light (high ISO film/settings, long shutter open times) while autofocus can be triggered by other items because it tends to focus on the brightest thing in view.

On the distal end of the scope can be the cheek retractor or other tissue manipulation device. In patients' mouths there are areas that are difficult to access by viewing alone. The cheek or tongue retractor enhances the ability to access such tissues, and gives the clinician a "third hand" - one to hold the scope, the second to hold the tongue and the "third" to retract the cheek. This can be particularly important if the clinician must work alone.

The retractor can be made up of a base and detachable arm, and can be configured to also function as a cap to prevent contamination. In certain embodiments, or for convenience, the retractor can have a plurality of positions around the end of the scope, for example eight circumferentially disposed positions that the retractor can be placed in should different angles be preferred. The retractor's arm(s) can be detachable so that when not needed it won't interfere with the procedure such as by poking the practitioner and/or patient. It may only needed to access about 10% of the mouth.

The retractor's arm(s) can be angled like a dental mirror. This can be designed to be similar with the other tools the practioner uses and to prevent slippage of the lips and/or cheek. It can be shaped such that only a small part of the retractor can be viewable to reduce occlusion of the viewing path while allowing the practitioner to know at all times where the tip is. The Ambient Light Management System (ALMS) is also used to block out ambient light.

The cheek retractor can have a ratcheting mechanism to allow adjustment of the cheek retractor angle without removing it from the device. Predetermined positions for the retractor can give defined positions for the device for documentation purposes, e.g., "when the device was at position NW, was able to see into the space...". The seal between the retractor and the rest of the VELScope can be purely frictional or with notches or grooves circumferentially about the device to hold it in place. Instead of eight positions, there could be a completely smooth ring on the VELScope allowing continuous positioning (infinite positions), for example if registration of the angle is not important. Instead of eight positions there could be six (e.g., with or without the top and bottom), four (e.g., if a square front was desired which could be useful with a square window), or any other number of positions.

In certain embodiments, the systems also comprise one or more of a detachable head for curing, and there can be a modular system with multiple heads for one light source, for example for curing at 476 nm, exciting fluorescence for cancer detection at 434 nm, and for exciting fluorescence for dental caries at 405 nm. The various components can be attached to each other in any desired manner, for example quick release, friction fits, screw on, etc. The head can contain different filters according to the task, for example diagnosis/treatment of caries/gingivitis.

For curing a combined viewing/illumination pathway (or indeed providing a viewing pathway) might not be desirable in certain embodiments because the practitioner may not need to be able to see the target, but could be of some derived benefit, for example because the illumination can, be filtered out to prevent the user from seeing it (typically harmful. UV light) so the filter on the light source can be for example a combination of a bandpass filter and a notch filter to pass a wide range of wavelengths including blue up to red and then a big notch in the middle to remove all the rest. In other words, expose in blue, view in red, but not necessarily for fluorescence viewing.

In certain embodiments, the systems also comprise a crosshair to point out where the viewer is looking, or have another laser or side light such as a dual LED to provide an aiming light. In various embodiments, the curing head can be a filter selecting the desired illumination wavelength and a curing head to direct the illumination. The curing head could be either permanently attached or a consumer off the shelf (COTS) version that could be removably attached to the filter head when required. The curing head could be standard, fiber optic, free space, light pipe, etc., any desired method of getting the light from the filter to the tissue.

As noted elsewhere, sometimes multiple light sources can be provided with a single scope. For white light viewing if desired, there could be provision for a greater bandwidth in the output. The larger bandwidth could be obtained by having an extra light (LED, halide, etc.) or by using different filters at the output of a single light source. The systems can also provide illumination with multiple peaks. For example, pharmacology/physiology testing of biological markers may sometimes use this for when fluorescence emitted (by the tissue, markers, or chemical signals) changes in the presence of various ions/molecules/pH. This can also be used to provide a normalization as the power of fluorescence produced by each wavelength can be being compared, normalized against each other. In certain embodiments, the systems also comprise timers to turn off the light for defined curing times or other purposes such as power savings.

In certain embodiments, the scope comprises a shockmounting to isolate the optical pathway in the VELScope. This can be for example a second exoskeleton wrapping around the first but separated from it by air and contacting at rubber o-ring shockmounts sandwiched in between the pathway tubing and the outer shell. This also presents a convenient way of changing the colour of the device without going to expensive overmolding techniques. The inner pathway is typically black for light absorption while black can be a colour not usually used in medical settings because of its depressing nature and how it looks dirty due to cleaning residue and powder from latex cloves. The outer shell can be a different colour such as white, beige, or other normally used colours to circumvent these issues.

In certain embodiments, the systems comprise a square window or port, which can be cheaper to make than a round window due to process manufacturing concerns, such as hole saws vs. straight line cutting, and reduced wastage. Moreover, such a configuration (or other non-round configuration) can assist the user to manipulate the ALMS by creating a friction/structural fit between the ALMS and the scope. The square window can also desirable for relatively inexpensive non-fluorescing glass/material in the optical pathway, although such material can also be used in other geometries.

The distal end of the VELScope can have a female port that can be square at the most distal end, but if desired the optical pathway remains circular. The port on the VELScope could be permanent or detachable to allow the use of differently shaped windows or the standard ALMS design. The shape of the ALMS can be such that there can be more material on the distal side of the patient from the practitioner. This reduces interference of the ALMS with the practitioner and takes advantage of the natural light blocking ability of the practitioner, and the ALMS can have a cutout for the nose in it.

In certain embodiments, the systems use weak-strength repositionable adhesives for "Post-it note" effect. The position can be adjusted if required and no residue or permanent attachment to the glasses. This allows reuse of the protective glasses and disposability of the contaminated ALMS. With the use of this adhesive, there can be also the possibility of presenting the ALMS in "sheets" combined into pads, where a new one can be peeled off as required. The size of the ALMS can cover both the scope and hand. The material can be black or other dark material to absorb reflected illumination and ambient light and to absorb emitted auto-fluorescence from the ALMS itself. It can be made from polypropylene or other desired material for cheapness and disposability. The weight can be selected to give draping without too much interference with the practitioner.

All terms used herein, are used in accordance with their ordinary meanings unless the context or definition clearly indicates otherwise. Also unless expressly indicated otherwise, the use of "or" includes "and" and vice-versa. Non-limiting terms are not to be construed as limiting unless expressly stated, or the context clearly indicates, otherwise (for example, "including," "having," and "comprising" typically indicate "including without limitation"). Singular forms, including in the claims, such as "a," "an," and "the" include the plural reference unless expressly stated, or the context clearly indicates, otherwise.

The scope of the present systems and methods, etc., includes both means plus function and step plus function concepts. However, the terms set forth in this application are not to be interpreted in the claims as indicating a "means plus function" relationship unless the word "means" is specifically recited in a claim, and are to be interpreted in the claims as indicating a "means plus function" relationship where the word "means" is specifically recited in a claim. Similarly, the terms set forth in this application are not to be interpreted in method or process claims as indicating a "step plus "function" relationship unless the word "step" is specifically recited in the claims, and are to be interpreted in the claims as indicating a "step plus function" relationship where the word "step" is specifically recited in a claim.

The innovations herein include not just the devices, systems, etc., discussed herein but all associated methods including methods of making the systems, making elements of the systems such as particular devices, such as the ALMS or the scopes, as well as methods of using the devices and systems, such as, for example, attaching the ALMSs to the scopes, and using the systems to interrogate a tissue (or otherwise using the scope to diagnose, treat, etc., a tissue). In some embodiments, the present innovations include methods of using the scope for both diagnosis and dental curing, for example the curing of cyanoacrylate-type glues used to attach or adhere dental prosthesis or other items into the oral cavity.

### EXAMPLE

### Example 1: Use Of A Viewing Scope To Identify Primary Dysplasia In The Mouth

Use of a viewing scope to identify primary dysplasia in the mouth (N=49) based on autofluorescence. The light path of the scope had in order a collimator, 430+/-30 nm filter (filter 1), a dichroic filter (filter 2), light to avoid absorber, glass window, mucosal tissue or other target tissue, dichroic filter (filter 2 (the light passes back past the same dichroic filter), 475 long pass filter (filter 3), 590 nm notch filter (filter 4), eyepiece ocular. The scope was used with a vestibular device with a port that operably linked to the scope and that blocked substantially all ambient light from reaching the target.

For primary lesions in patients with no history of oral cancer, the scope correctly identified the majority of dysplasia (70%), particularly high-grade preinvasive lesions (86%). About 18% of non-dysplastic lesions were also positive using the scope. The scope also improved the ability of the clinician to differentiate non-dysplastic lesions. Nine of eleven such non-dysplastic lesions were clinically diagnosed as oral premalignant lesions using standard techniques, but only two of these false positives were also positive using the scope.

In some other results, ten biopsies from lesions identified as positive using the scope that were actually dysplastic also had biopsies from adjacent normal looking areas that were properly identified using the scope as non-dysplastic.

## Claims

1. A viewing scope (104) for a human viewer to view a target tissue of a patient, the viewing scope comprising a hollow casing comprising a proximal end (192), a distal end (190) at least about one inch in diameter and a light source configured to shine light on the target tissue, the scope configured to selectively collect at least fluorescent light emanating from the target tissue and transmit the light through the hollow casing to selectively provide substantially only fluorescent light to an ocular eye piece (145) located at or about the proximal end of the hollow casing, wherein the viewing scope is sized and configured to be wireless and hand held and wherein the viewing scope is substantially T-shaped such that a handle (198) extends substantially downwardly from the hollow casing, and the viewing scope further comprises a battery (266) operably connected to the light source to provide power to the light source.

2. The viewing scope of claim 1 wherein the distal end of the viewing scope further comprises a distally located light source disposed in a ring around or about the distal end of the hollow casing and configured to selectively shine substantially only fluorescence-inducing blue excitation light on the target tissue.

3. The viewing scope of any of the previous claims wherein the distally located light source is configured to selectively provide the substantially only fluorescence-inducing blue excitation light and is also configured to shine white light on the target tissue.

4. The viewing scope of any of the previous claims, wherein the distal end of the viewing scope further comprises a removable, substantially non-fluorescing, non-reflective window (106) that substantially covers the distal tip of the viewing scope

5. The viewing scope of any of the previous claims, wherein the distally located light source comprises a plurality of light emitting diodes disposed substantially in a circle and configured to emit substantially only blue light.

6. The viewing scope of any of the previous claims wherein the viewing scope further comprises a high-ISO imaging sensor configured to image fluorescent light emanating from the target tissue.

7. The viewing scope of any one of the previous claims wherein the window comprises an optical filter configured to selectively block undesirable wavelengths of light and transmit desired wavelengths of light.

8. The viewing scope of any one of the previous claims wherein the window is held within a port ring (302) that removably attaches at or about the distal end of the hollow casing.

9. The viewing scope of any one of the previous claims wherein the viewing scope further comprises a light source configured to provide an illumination light path that shines light on the target tissue, the scope configured to selectively collect at least fluorescent light emanating from the target tissue and transmit the light along a receiving light path (142) through the hollow casing to selectively provide substantially only fluorescent light to an ocular eye piece located at or about the proximal end of the hollow casing, wherein the viewing scope comprises at least one of a collimating optical element (234) or a focusing optical element and the light transmitted to the target tissue is at least one of collimated light or focused light.

10. The viewing scope of claim 9 wherein the viewing scope further comprises a diffusing optical element such that the light transmitted to the target tissue has a substantially uniform intensity across the light beam.

11. The viewing scope of claim 9 or 10 wherein the light source is configured to selectively provide varying wavelengths of light.

12. The viewing scope of claims 9 to 11 wherein the viewing scope is configured such that the illumination light path and the collection light path are co-linear.

13. The viewing scope of claim 12 wherein the illumination light path and the collection light path follow the same path for at least a portion of each of the light paths.
